# EUROPEAN PATENT APPLICATION

(11) **EP 4 074 329 A1**
(43) Date of publication of application: **19.10.2022**
(21) Application number: 20899627.2
(22) Date of filing: 10.12.2020
(51) Int. Cl.: A61K 38/17, A61K 35/39, A61K 48/00, A61P 3/10, C12N 5/10, C12N 15/12, C12N 15/63

(54) **INDUCTION OF PROLIFEROUS PANCREATIC ISLET PRECURSOR CELL-LIKE CELLS BY TRANSIENT EXPRESSION OF MYCL AND INDUCTION OF DIFFERENTIATION INTO INSULIN-POSITIVE CELLS**

(30) Priority: 11.12.2019 JP 2019223953
(71) Applicant: The University of Tokyo, Bunkyo-ku, Tokyo 113-8654 (JP)
(72) Inventor: YAMADA, Yasuhiro, Tokyo 113-8654 (JP); HIRANO, Michitada, Tokyo 113-8654 (JP)
(74) Representative: advotec.
(86) International application number: PCT/JP2020/046177
(87) International publication number: WO 2021/117840

(57) **Abstract**

Establishment of pancreatic islet-like insulin producing cells by inducing differentiation of ES/iPS cells has been reported. However, no technique has been developed so far for producing functional pancreatic islet insulin-positive cells in a large amount. In addition, there are concerns regarding rejection responses, accidental immune responses, etc. The present invention provides pancreatic islet-like cells having Mycl gene introduced thereinto and a method that comprises inducing proliferation of pancreatic islet-like cells by transient expression of Mycl gene and then inducing degradation thereof into insulin producing cells.

## Description

### FIELD

The present invention relates to an insulin production promoter that includes the Mycl gene or pancreatic islet-like cells having the Mycl gene introduced therein. The invention further relates to a pharmaceutical composition for preventing or treating diabetes, which includes the Mycl gene, its gene product, or pancreatic islet-like cells having the Mycl gene introduced therein. The invention still further relates to a method for inducing differentiation of insulin-producing cells in which proliferous pancreatic islet precursor cell-like cells are induced by transient expression of the Mycl gene.

### BACKGROUND

It has been shown that when brief expression of cell reprogramming factors is repeated in a mouse individual, pancreatic islet cells proliferate, improving glucose tolerance (NPL 1).

Pancreatic islet transplantation is a type of medical care whereby blood sugar can be stabilized by grafting of pancreatic islet tissue into a diabetes patient, but due to the serious problem of a lack of donors in Japan, there is an urgent need to develop pancreatic islet function regeneration methods based on different approaches. Establishment of pancreatic islet-like insulin-producing cells by inducing differentiation of ES/iPS cells has recently been reported, but a technique for producing large amounts of functional pancreatic islet insulin-positive cells still has not yet been devised, while the problems of rejection and autoimmune reaction remain a concern.

### [CITATION LIST]

### [NON PATENT LITERATURE]

[NPL 1] Cell, 2016,167,1719-1733, e12. doi: 10/1016/j. cell. 2016. 11. 052

### SUMMARY

### [TECHNICAL PROBLEM]

Techniques for growing pancreatic islet insulin-producing cells by transient expression of the Mycl gene are expected to allow establishment of large amounts of functional pancreatic islet insulin-producing cells from small volumes of donor-derived pancreatic islets. It is also expected that insulin-positive cells can be efficiently produced from stem cells by applying such techniques during the course of inducing pancreatic islet insulin cells from ES/iPS cells. It is an object of the present invention to provide means for treating diabetes, which means includes removing pancreatic islet cells from a subject, and returning the pancreatic islet cells back to the subject after inducing proliferous pancreatic islet precursor cell-like cells *in vitro.* It is a further object of the invention to provide means for treating diabetes by transient overexpression of the Mycl gene in pancreatic islet cells in the body.

### [SOLUTION TO PROBLEM]

The present inventors have found that proliferous pancreatic islet precursor cell-like cells can be induced by introducing the Mycl gene into pancreatic islet cells and expressing the gene. In the presence of the Mycl gene, the proliferating cells express the Fev gene, Pax4 gene and Cck gene which are expressed in embryonic pancreatic islet precursor cells, while also simultaneously expressing somatostatin and proliferating, but when Mycl gene expression stops the proliferation ceases as differentiation to insulin-positive cells takes place. In other words, it was found that inducing transient expression of the Mycl gene can induce proliferous pancreatic islet precursor cell-like cells, and that the proliferated pancreatic islet precursor cell-like cells can differentiate to insulin-positive cells, and the present invention has been completed on the basis of this finding.

Specifically, the invention has the following aspects.
[1] An insulin production promoting agent that includes the Mycl gene or its gene product.
[2] The insulin production promoting agent according to [1] above, wherein the Mycl gene includes:
   (1) a nucleic acid including the nucleotide sequence represented by SEQ ID NO: 1 or 3; or
   (2) a nucleic acid that hybridizes with a nucleic acid including the nucleotide sequence represented by SEQ ID NO: 1 or 3 under stringent conditions and encodes a polypeptide having activity of promoting insulin production when its expression is induced by the Mycl gene.
[3] The insulin production promoting agent according to [1] above, wherein the Mycl gene product includes:
   (1) a polypeptide including the amino acid sequence represented by SEQ ID NO: 2 or 4; or
   (2) a polypeptide having at least 80%, 85%, 90%, 95%, 97%, 98% or 99% sequence identity with the amino acid sequence represented by SEQ ID NO: 2 or 4, and having an effect of causing proliferation of pancreatic islet-like cells and/or activity of promoting insulin production.
[4] An insulin production promoting agent, having the Mycl gene or its gene product introduced into pancreatic islet cells.
[5] The insulin production promoting agent according to any one of [1] to [4] above, wherein the Mycl gene is transiently expressed.
[6] The insulin production promoting agent according to [4] or [5] above, wherein the pancreatic islet cells are derived from primary pancreatic islet cells isolated from a pancreas, cultured pancreatic islet cells or stem cells.
[7] The insulin production promoting agent according to [6] above, wherein the stem cells are selected from the group consisting of iPS cells, ES cells and somatic stem cells.
[8] A pharmaceutical composition for prevention and/or treatment of diabetes or its associated disease, the pharmaceutical composition including:
   (i) the Mycl gene or its gene product; a vector in which the Mycl gene has been incorporated; and/or pancreatic islet cells in which the Mycl gene or its gene product has been introduced or its expression has been induced, and
   (ii) a pharmaceutically acceptable excipient, diluent or carrier.
[9] The pharmaceutical composition according to [8] above, wherein the diabetes or its associated disease is selected from among diseases, disorders or symptoms associated with type I diabetes, type II diabetes, impaired glucose tolerance, hyperglycemia, heterolipidemia, obesity and metabolic syndrome.
[10] The pharmaceutical composition according to [8] or [9] above, wherein the Mycl gene includes:
   (1) a nucleic acid including the nucleotide sequence represented by SEQ ID NO: 1 or 3; or
   (2) a nucleic acid that hybridizes with a nucleic acid including the nucleotide sequence represented by SEQ ID NO: 1 or 3 under stringent conditions and encodes a polypeptide having activity of promoting insulin production when its expression is induced by the Mycl gene.
[11] The pharmaceutical composition according to [8] or [9] above, wherein the Mycl gene product includes:
   (1) a polypeptide including the amino acid sequence represented by SEQ ID NO: 2 or 4; or
   (2) a polypeptide having at least 80%, 85%, 90%, 95%, 97%, 98% or 99% sequence identity with the amino acid sequence represented by SEQ ID NO: 2 or 3, and having an effect of causing proliferation of pancreatic islet-like cells and/or activity of promoting insulin production.
[12] A kit including an insulin production promoting agent according to any one of [1] to [7] above or a pharmaceutical composition according to any one of [8] to [11] above.
[13] A kit further including an activator for activation of the Mycl gene.
[14] The kit according to [13] above, wherein the activator for activation of the Mycl gene is selected from the group consisting of promoters, enhancers, promoter-activating enzymes or factors, enhancer-activating enzymes or factors, nucleic acid-protein complexes and low molecular compounds.
[15] Pancreatic islet cells in which the Mycl gene or its gene product has been introduced.
[16] The pancreatic islet-like cells according to [15] above, wherein the pancreatic islet cells are derived from primary pancreatic islet cells isolated from a pancreas, cultured pancreatic islet cells or stem cells.
[17] A method for preparing pancreatic islet-like cells according to [16] above, the method including:
   (a) a step of incorporating the Mycl gene into a recombination plasmid, recombination viral vector, minicircle or episomal vector; and
   (b) a step of introducing the recombination plasmid, recombination viral vector, minicircle or episomal vector obtained in step (a) into pancreatic islet cells.
[18] A method for preparing pancreatic islet-like cells according to [16] above, the method including a step of introducing RNA encoding the Mycl gene, or Mycl protein, into pancreatic islet cells.
[19] A method for proliferating pancreatic islet-like cells according to [15] or [16] above, or pancreatic islet-like cells prepared by the method of according to [17] or [18] above, the method including a step of expressing the Mycl gene.
[20] The method according to [19] above, wherein expression of the Mycl gene is transient.

### [ADVANTAGEOUS EFFECTS OF INVENTION]

According to the invention it is possible to treat diabetes or its associated disease in which insulin production is desired, by controlling exogenous or endogenous Mycl gene expression.

### BRIEF DESCRIPTION OF DRAWINGS

Fig. 1 shows establishment of mouse ES cells in which Mycl expression can be induced.
Fig. 2 shows increase in pancreatic islets by Mycl overexpression.
Fig. 3 shows hyperplasia of somatostatin-positive pancreatic islet cell-like cells by Mycl overexpression.
Fig. 4 shows hyperplasia of somatostatin-positive pancreatic islet cell-like cells by Mycl overexpression.
Fig. 5 shows hyperplasia of somatostatin-positive pancreatic islet cell-like cells by Mycl overexpression.
Fig. 6 shows the similarity of hyperplasic somatostatin-positive pancreatic islet cell-like cells produced by Mycl overexpression, with embryonic pancreatic islet precursor cells.
Fig. 7 shows proliferation induction of pancreatic islet precursor cell-like cells by Mycl overexpression, and proliferation cessation by cessation of Mycl overexpression.
Fig. 8 shows differentiation of pancreatic islet precursor cell-like cells into insulin-positive cells by cessation of Mycl expression.
Fig. 9 shows enhancement of glucose tolerance by transient Mycl overexpression.
Fig. 10 shows induction of Mycl expression and increase in pancreatic islets *in vitro* for isolated pancreatic islets.
Fig. 11 shows induction of Mycl expression and proliferation of pancreatic islet cells *in vitro,* for cells dispersed from isolated pancreatic islets.
Fig. 12 shows establishment of mouse ES cells in which c-Myc gene or Mycn gene expression can be induced.
Fig. 13 shows cell death by c-Myc gene or Mycn gene expression *in vitro,* for isolated pancreatic islets.
Fig. 14 shows the therapeutic effect in a mice diabetes model.
Fig. 15 shows that Mycl does not induce abnormal proliferation other than in pancreatic islets.
Fig. 16 shows hyperplasia of pancreatic islets in aged mice.
Fig. 17 shows Mcyl expression in human pancreatic islet precursor cells.
Fig. 18 shows a diagram confirming hyperplasia of pancreatic islets in a human.
Fig. 19 shows the results of confirming hyperplasia of pancreatic islets in a human based on single cell analysis.

### DESCRIPTION OF EMBODIMENTS

The present invention relates to an insulin production promoting agent that includes the Mycl gene or its gene product, or pancreatic islet-like cells having the Mycl gene introduced therein. It further relates to a pharmaceutical composition for prevention or treatment of diabetes, which includes the insulin production promoting agent. The present invention will now be explained in greater detail.

### 1. Pancreatic islet-like cells and preparation method

### (1) Pancreatic islet-like cells

The term "pancreatic islet-like cells" as used herein refers to pancreatic islet cells in which the Mycl gene or its gene product has been introduced. Throughout the present specification, pancreatic islet-like cells that have moved to the growth phase by forced expression of the Mycl gene will be referred to as "pancreatic islet precursor cell-like cells". Stopping expression of the gene can cause differentiation of cells that are able to produce insulin (hereunder also referred to as "insulin-producing cells"). In other words, the present invention increases the number of pancreatic islet-like cells by transient expression of the Mycl gene, and causes them to differentiate into cells that produce insulin. Focusing on markers expressed by different cells, "pancreatic islet precursor cell-like cells" are positive for at least one, preferably two and more preferably three or more genes selected from among Fev, Pax4, Cck, CDK4 and Ki67, for example. Focusing on the expressed proteins, they are characterized by reduced production of insulin and glucagon in particular compared to normal pancreatic islets, or by notable somatostatin production.

### (2) Pancreatic islet cells

"Pancreatic islet cells" generally refers to cell aggregates known as the islet of Langerhans which perform pancreatic endocrine functions, as the endocrine cells making up about 1 to 2% of all of the cells in the pancreas. Pancreatic islet cells are largely composed of five different types of cells: α cells, β cells, δ cells, ε cells and PP cells. The major cells composing the cell aggregates are β cells, occupying the center of the pancreas. The β cells constitute about 60 to 80% of the cell aggregates and secrete insulin which aids in migration of glucose into most cells in the body. The α cells, on the other hand, make up about 10 to 30% of pancreatic islets and secrete glucagon which is released during fasting, and aid in release of glucose from the liver to maintain normal blood sugar. The δ cells make up about 5 to 10% of pancreatic islet cells and secrete somatostatin which further adjusts glucose levels. The ε cells and PP cells secrete ghrelin and pancreatic polypeptides, respectively. Pancreatic polypeptide-producing cells (about 5 to 10% of pancreatic islet cells) release hormones which alter exocrine and gastrointestinal functions. Pancreatic islet cells also include other types such as endothelial cells, neurons and progenitor cells.

The term "pancreatic islet cells" as used herein includes these pancreatic islet cells and pancreatic islet precursor cells which are progenitors of pancreatic islet cells, and they may also be intermediate cells that develop on the way to pancreatic islet cells or pancreatic islet precursor cells, produced during the course of development into pancreatic islet cells or during the course of inducing differentiation from somatic stem cells or pluripotent stem cells. The term "intermediate cells" preferably refers to cells whose destiny for differentiation to pancreatic islet cells has been determined. According to the invention, pancreatic islet cells may be created from pancreatic islet-like cells or pancreatic islet precursor cell-like cells in which the Mycl gene or its gene product has been introduced.

In type I diabetes, cellular infiltration mainly of lymphocytes is seen soon after onset. This eventually leads to selective loss of β cells, and reduction in pancreatic islet volume, leaving primarily α cells. Pancreatic islets are capable of reserve insulin secretion, and loss of 90 to 95% of β cells is onset of type I diabetes. Basically no morphological changes are seen in the pancreatic islets in type II diabetes, which is associated only with reduced insulin secretion by the pancreatic islets.

According to the invention, the origin or source of pancreatic islet cells into which the Mycl gene or its gene product is introduced is not restricted and may be primary pancreatic islet cells isolated from the pancreas of an individual or pancreatic islet cells cultured by a known culturing method. The cultured pancreatic islet cells are not limited, and may include stocked pancreatic islet cells, or pancreatic islet cells derived from stem cells (such as iPS cells, ES cells or somatic stem cells) (see Kimura, A., et al., Cell Chemical Biology, 2020, doi.org/10.1016/j.chembiol.2020.08.018, for example). The pancreatic islet cells into which the Mycl gene or its gene product is introduced according to the invention may also be pancreatic islet cells obtained from pancreatic islet-like cells and/or pancreatic islet precursor cell-like cells, and the Mycl gene or its gene product may also be again introduced into pancreatic islet-like cells and/or pancreatic islet precursor cell-like cells into which the Mycl gene has been introduced. Both pancreatic islet cells harvested from a donor and pancreatic islet cells derived from stem cells are suitable for the purpose of treating diabetes. Pancreatic islet cells from a donor may be either autologous or heterologous with respect to the recipient. According to the invention, introduction of the Mycl gene into pancreatic islet cells to cause proliferation of pancreatic islet precursor cell-like cells *in vitro,* and returning the cells (grafting) into a patient, can be used for treatment of diabetes. When the pancreatic islet cells are heterologous with respect to the patient, the patient may also be administered an immunosuppressive agent as appropriate. Grafting into the patient may be with pancreatic islet-like cells, pancreatic islet precursor cell-like cells, insulin-producing cells, or any desired combination thereof.

For loss of β cells in mice at least, previous reports have been published in regard to proliferating α cells and differentiating a portion of the α cells into β cells. Pancreatic islets used as a source of pancreatic islet cells into which the Mycl gene is to be introduced may therefore be normal pancreatic islets containing large numbers of β cells, or pancreatic islets which have lost most of their β cells. From this viewpoint the present invention can also be applied for gene therapy of type I diabetes patients with loss of β cells, using pancreatic islet-like cells into which the Mycl gene has been introduced.

### (3) Pluripotent stem cells

The term "pluripotent stem cells" used herein refers to cells with self-replicating ability and pluripotency, the cells having the ability to form different types of cells composing the body. The term "self-replicating ability" means the ability to produce identical undifferentiated cells from a single cell. The term "differentiation potency" means the ability for a cell to differentiate. Examples of pluripotent stem cells include embryonic stem cells (ES cells), induced pluripotent stem cells (iPS cells), Muse cells (Multi-lineage differentiating Stress Enduring cells), germline stem cells (GS cells) and embryonic germ cells (EG cells), with no limitation to these. The pluripotent stem cells used for the invention are preferably ES cells. Pluripotent stem cells may be from a mammal, bird, fish, reptile or amphibian, with no particular limitations. Mammals include primates (humans, monkeys, etc.), rodents (mice, rats, guinea pigs, etc.), cats, dogs, rabbits, sheep, pigs, cows, horses, donkeys, goats and ferrets.

The term "ES cells" used herein refers to pluripotent stem cells having the ability to differentiate into all tissue cells of the body during early development, which are stocked so as to be taken and cultured *in vitro.* ES cells can be increased essentially indefinitely while retaining the ability to differentiate into all cells of the body similar to pluripotent stem cells of the early embryo. Specifically, mouse ES cells were first described in 1981 (Proc. Natl. Acad. Sci. USA 78,7634-7638,1981; Nature 292,154-156,1981). ES cells are pluripotent and can generate all tissues and cell types of the body. Pluripotent embryonic stem cells have been isolated from numerous and various species including rat (Iannaconns et al., Dev. Biol. 163, 288-292, 1994), hamster (Dev. Biol. 127, 224-227, 1988), rabbit (Mol. Reprod. Dev. 36, 424-433, 1993), bird, fish and pig (Reprod. Fertil. Dev. 6, 563-568, 1994), cow (Reprod. Fertil. Dev. 6, 553-562, 1994) and primate (Proc. Natl. Acad. Sci. USA 92, 7844-7848, 1995). ES cells to be used for the invention are not restricted and may be KH2 cells, RF8 cells, JI cells, CGR8 cells, MG1.19 cells, 129SV cells, C57/BL6 cells, or DBA-1 cells.

Several research teams have successfully isolated ES cells and ES cell-like stem cells from embryonic human tissue. The initial success was as described in Science 282, 1145-1147,1998; Proc. Natl. Acad. Sci. USA 95, 13726-13731, 1998; Nature Biotech., 18, 399-404, 2000. These ES cell lines were established by culturing ICM isolated from blastocysts on feeder cells. Other recent research indicates that nuclei from embryos and mature mammalian cells can be grafted into enucleated oocytes to obtain embryos and embryonic cells.

According to the invention it is possible to use any established ES cell line. Alternatively, it is effective to create a clone embryo using somatic cells from an individual and to establish an ES cell line from it, in order to prevent immunorejection that occurs when ES cells prepared by the method of the invention are used in an individual. Using this method allows ES cells to be established that have the same genetic elements as the individual.

Alternatively, the "reprogramming" phenomenon may be used, whereby somatic cell nuclei introduced into eggs when creating somatic cell clones change into the same state as fertilized ovum nuclei. It has also been reported that ES cells have activity similar to this type of activity of eggs (Curr. Biol., 11, 1553-1558, 2001). In other words, fusion of individual somatic cells and ES cells is expected to allow somatic cells to be converted to cells such as ES cells. Since ES cells can be genetically engineered *in vitro,* carrying out the procedure using ES cells with prior manipulation of the factors associated with immunological rejection, such as the MHC gene group, makes it potentially possible to avoid rejection without using a method such as somatic cell clone embryo creation.

As used herein, the term "induced pluripotent stem (iPS) cells" refers to cells with pluripotent differentiating power similar to ES cells, obtained by introducing genes for transcription factors such as Oct3/4, Sox2, Klf4 or c-Myc into somatic cells. It is likewise possible to indefinitely increase iPS cells that retain pluripotent differentiating power, similar to ES cells.

One basic method for creating iPS cells involves using viruses for introduction of the four transcription factors Oct3/4, Sox2, Klf4 and c-Myc into cells (Takahashi K, Yamanaka S: Cell 126(4), 663-676, 2006; Takahashi, K, et al: Cell 131(5), 861-72, 2007). Examples of cells to be used for creating iPS cells, i.e. cells from which iPS cells can be derived, include lymphocytes (T cells and B cells), fibroblasts, epithelial cells, endothelial cells, mucosal epithelial cells, mesenchymal stem cells, hematopoietic stem cells, adipose stem cells, dental pulp stem cells and neural stem cells.

Reprogramming of iPS cells can be carried out by a method known to those skilled in the art, such as Addgene's Blog/Post, "Delivery Methods for Generating iPSCs" (https://blog.addgene.org/delivery-methods-for-generating-ipscs). The method for introducing the Mycl gene into iPS cells is not restricted and may be a method of introduction using a recombinant virus (such as retrovirus, lentivirus, adenovirus or Sendai virus), a recombination plasmid, minicircle or episome (such as oriP/Epstein-Barr nuclear antigen-1 (EBNA1) episomal vector), or a method of introducing RNA (including mRNA) encoding the Mycl gene or the Mycl protein itself directly into cells.

The term "EG cells" as used herein refers to any embryonic germ cells created from primordial germ cells, with no particular restriction on their source. Also as used herein, "GS cells" refers to a cell line of germ line cells created from testes germ cells, which allow spermatic stem cells (germline stem cells) to be cultured *in vitro* (Cell. 119, 1001-1012, 2004). Preferred among GS cells are mGS cells (multipotent germline stem cells) that have pluripotent differentiating power and a nature similar to that of ES cells.

### (4) Mycl gene and its gene product

The Mycl (also "L-Myc") gene is a member of the Myc gene family which includes the c-Myc gene and Mycn ("N-Myc") gene. The Mycl gene is an oncogene similar to the c-Myc gene, and is known as a reprogramming gene. The Mycl gene also differs from the c-Myc gene in having virtually no transformation ability (Nakagawa, M., et al., Proc. Natl. Acad. Sci. USA, vol. 107, p. 14152-14157, 2010). The cDNA sequence information for mouse and human Mycl can be obtained by referring to NCBI Accession No. NM_008506 and NM 001033081, respectively, and a person skilled in the art can easily isolate the cDNA.

According to the invention it is preferable to use the isolated Mycl gene and gene product. As mentioned above, the nucleotide sequence of the Mycl gene can be identified based by NCBI Accession No., and usable Mycl genes include single-stranded or doublestranded DNA, and their RNA complements. DNA includes naturally-derived DNA, recombinant DNA, chemically synthesized DNA, DNA amplified by PCR, and combinations of the foregoing. DNA is preferred as the nucleic acid to be used for the invention. As is well known, the genetic code is degenerate, with some amino acids having multiple nucleotide sequences coding for the same amino acid, and there are no particular restrictions so long as the pancreatic islet cells into which the Mycl gene has been introduced (pancreatic islet-like cells) undergo cell proliferation by expression of the Mycl gene, and exhibit accelerated insulin production when its expression is stopped.

According to one embodiment, the Mycl gene may include or consist of:
(1) nucleic acid including or consisting of the nucleotide sequence represented by SEQ ID NO: 1 or 3; or
(2) nucleic acid that hybridizes with nucleic acid including or consisting of the nucleotide sequence represented by SEQ ID NO: 1 or 3 under stringent conditions and encoding a polypeptide having activity of causing proliferation of pancreatic islet-like cells in which the Mycl gene has been introduced; or
(3) nucleic acid that hybridizes with nucleic acid including or consisting of the nucleotide sequence represented by SEQ ID NO: 1 or 3 under stringent conditions and encoding a polypeptide having an effect of causing proliferation of insulin-producing cells as a result of causing proliferation of pancreatic islet-like cells into which the Mycl gene has been introduced, and thus causing acceleration of insulin production.

The phrase "under stringent conditions" as used herein means hybridizing under moderately or highly stringent conditions. Specifically, moderately stringent conditions can be easily determined by a person skilled in the art with access to common technology, based on the DNA length. The basic conditions are described by Sambrook, J. et al. in Molecular Cloning, A Laboratory Manual (3rd edition), Cold Spring Harbor Laboratory, 7.42-7.45(2001), but for a nitrocellulose filter, and a pre-rinsing solution of 5 × SSC, 0.5% SDS, 1.0 mM EDTA (pH 8.0), the conditions used may be hybridization conditions with ~50% formamide, 2 × SSC to 6 × SSC at about 40 to 50°C (or another similar hybridization solution such as Stark's solution in ~50% formamide at about 42°C), and rinsing conditions with 0.5 × SSC, 0.1% SDS at about 60°C. Highly stringent conditions can also be easily determined by a person skilled in the art based on the DNA length, for example. Such conditions are generally hybridization and/or rinsing with a higher temperature and/or lower salt concentration than moderately stringent conditions, and for example, such hybridization conditions are defined as rinsing with 0.2 × SSC, 0.1% SDS at about 68°C. A person skilled in the art will appreciate that the temperature and rinsing solution salt concentration can be adjusted as necessary depending on factors such as probe length.

Homologous nucleic acid cloned using nucleic acid amplification reaction or hybridization has 30% or greater, preferably 50% or greater, more preferably 70% or greater, even more preferably 90% or greater, yet more preferably 95% or greater and most preferably 98% or greater identity with the respective nucleotide sequences listed as SEQ ID NO: 1 or 3. The percent identity can be determined by visual examination and mathematical calculation. Alternatively, the percent identity of two nucleic acid sequences can be determined by comparing sequence information using the GAP computer program mentioned in Devereux et al., Nucl. Acids Res., 12, 387(1984) and available from University of Wisconsin, Genetic Computer Group (UWGCG) (GCG Wisconsin Package, version 10.3).

According to one embodiment, the gene product of the Mycl gene is the polypeptide expressed by the Mycl gene. The polypeptide may typically be:
(1) a polypeptide including or consisting of the amino acid sequence represented by SEQ ID NO: 2 or 4; or
(2) a polypeptide having at least 80%, 85%, 90%, 95%, 97%, 98% or 99% sequence identity with the amino acid sequence represented by SEQ ID NO: 2 or 4, and having an effect of causing proliferation of pancreatic islet-like cells and/or activity of promoting insulin production.

According to one embodiment, the gene product of the Mycl gene may be a mutant polypeptide as defined above, and may be the amino acid sequence of SEQ ID NO: 2 or 4 having a deletion, substitution, insertion and/or addition of one or more amino acids. A substitution may be a conservative substitution, which is a substitution of specific amino acid residues with residues having similar physicochemical properties. Non-limitative examples of conservative substitutions include substitutions among aliphatic group-containing amino acid residues, such as mutual substitutions of Ile, Val, Leu and Ala, and substitutions among polar residues, such as mutual substitutions of Lys and Arg, Glu and Asp or Gln and Asn.

Mutations by deletion, substitution, insertion and/or addition of amino acids can be produced by the well-known technique of site-directed mutagenesis (Nucleic Acid Research, Vol. 10, No. 20, p.6487-6500, 1982, for example) of the Mycl gene. As used herein, the term "one or more amino acids" means a number of amino acids that can be deleted, substituted, inserted and/or added by a site-directed mutagenesis method. The term "one or more amino acids" used herein may also refer merely to one or several amino acids.

The method of deleting, substituting, inserting and/or adding one or more amino acids to the amino acid sequence of a polypeptide while retaining its activity may also be, instead of the aforementioned site-directed mutagenesis, a method of treating the gene with a mutation source, or a method of selectively cleaving the gene and then removing the selected nucleotide and then making a substitution, insertion or addition followed by linkage. Without being limitative, the gene product of the Mycl gene of the invention may be a polypeptide having an amino acid sequence with a deletion, substitution or addition of 1 to 10, preferably 9 or fewer, 7 or fewer, 5 or fewer, 3 or fewer or 2 or fewer, and more preferably 1 amino acid in SEQ ID NO: 2 or 4, and having activity of promoting insulin production.

The mutant is a protein comprising an amino acid sequence having at least 80% or greater, preferably 85% or greater, 90% or greater, 95% or greater, 96% or greater, 97% or greater, 98% or greater or 99% or greater amino acid identity with the amino acid sequence listed as SEQ ID NO: 2, the polypeptide having an effect of causing proliferation of pancreatic islet-like cells and/or an effect of promoting insulin production.

The percent identity between two amino acid sequences can be determined by visual examination and mathematical calculation. Alternatively, the percent identity between two protein sequences can be determined by comparing sequence information using the GAP computer program available from University of Wisconsin, Genetic Computer Group (UWGCG), based on the algorithm described in Needleman, S.B. and Wunsch, C.D. (J. Mol. Biol., 48:443-453, 1970). The preferred default parameters for the GAP program include: (1) Scoring matrix, blosum62, as described in Henikoff, S. and Henikoff, J.G. (Proc. Natl. Acad. Sci. USA, 89:10915-10919, 1992), (2) 12 gap weight, (3) 4 gap weight; and (4) no penalty for end gap.

### (5) Introduction of Mycl gene

According to the invention, the method of introducing the Mycl gene into primary pancreatic islet cells, cultured pancreatic islet cells or stem cells (such as iPS cells, ES cells and somatic stem cells) is not particularly restricted, and any method publicly known to those skilled in the art may be used. Common gene transfer means are "transformation" and "transfection", which refer to transient or stable genetic changes induced in cells after incorporating exogenous nucleic acid (such as exogenous DNA or RNA for the host cells). Genetic changes can be obtained by incorporating exogenous nucleic acid into the genome of host cells, or by transiently or stably maintaining exogenous nucleic acid either as an episome component or independently. According to the invention, the introduced Mycl gene may be incorporated into the host cell genome so long as it is possible to control on/off switching of the gene expression, or it may be present as an episome component, or it may be present in the cytoplasm as a plasmid or vector containing the gene.

A "vector" is commonly used for introduction of exogenous nucleic acid (preferably DNA) into host cells. Vectors commonly include viruses, and particularly attenuated viruses and/or non-replicatable viruses. A viral vector may be a retrovirus vector, a lentivirus vector, an adenovirus vector, an adeno-associated virus vector or a Sendai virus vector. The vector may also include a regulatory sequence such as a promoter, enhancer, ribosome-binding sequence, terminator or polyadenylated site, to allow exogenous nucleic acid expression. If necessary, it may also include a selective marker sequence such as a drug resistance gene (a kanamycin resistance gene, ampicillin resistance gene or puromycin resistance gene, for example), a thymidine kinase gene or a diphtheria toxin gene, or a reporter gene sequence such as a fluorescent protein, β-glucuronidase (GUS) or FLAG. The term "promoter" used here is intended to include promoter components that are sufficient for cell type-specific controllable, tissue-specific controllable or exogenous signal- or chemical agent-inducible promoter-dependent gene expression. Such components may be located at the 5'-region or 3'-region of a natural gene. The term "functionally linked" means that a DNA sequence and regulatory sequence are linked in such a manner as to allow expression when a suitable molecule (such as a transcription activation protein) binds to the regulatory sequence.

Introduction of the vector into the host cells is not particularly restricted and may be carried out by the electroporation method (Meiner, V. et al., Proc. Natl. Acad. Sci. USA, 93:14041-14046(1996)), the calcium phosphate method, the DEAE-dextran method or a method using gene transfer lipids (lipofectamine or lipofectin). The vector-transferred cells may then be selected based on the property of a marker gene (such as a drug resistance gene). This can be confirmed by Southern blotting in which the probe used is a portion of exogenous nucleic acid that is the target of proper homologous recombination in the selected cells. It is thus possible to create cells containing a heterologous gene comprising the target gene, and specifically the Mycl gene, with knock-in of the marker gene.

The ES cells used may be the KH2 line having the Frt sequence downstream from the Cola1 gene locus, and expressing the reverse tetracycline regulatory transactivator M2-rtTA under the control of the endogenous Rosa26 promoter (Beard C, et al., Genesis, vol.44, p.23-28(2006)). Introduction of the Mycl gene into the ES cells can be carried out using a method known to those skilled in the art, and for example, pCR8-GW-TOPO vector (Invitrogen Life Technologies) which is an entry vector, may be inserted by TA cloning of the Mycl gene, and then LR reaction may be carried out between this vector and the Colla1-TetOP-AttR1-ccdB-AttR2-ires-mCherry vector, for example, and the TetOP-Mycl-ires-mCherry vector used as a gene transfer vector. The "TetOP (operon)" sequence in the vector is a sequence to which the reverse tetracycline regulatory transactivator binds (tetracycline response element: TRE), and it binds with a reverse tetracycline regulatory transactivator expressed from host cells in a manner dependent on reverse tetracycline, such as doxycycline (Dox) added to the cells, inducing expression of the gene linked downstream from it. The "ires" (internal ribosome entry site) is a ribosome internal recognition sequence, while "mCherry" is a gene coding for red fluorescent protein (reporter gene). By introducing the vector into KH2-ES cells together with nucleic acid coding for flipase, it is possible to incorporate the Mycl gene into ES cell chromosomes. In addition to the vector including "ires-mCherry", a similar vector may be used which contains pBSSK(-)-IRES-βgeo comprising a "ires-βgeo cassette" containing resistance genes (fused β-galactosidase and neomycin resistance genes) (Mountford P. et al., Proc. Natl. Sci. USA, 91:4303-4307(1994) , and IRES-Hygro (hygromycin resistance gene) cassette.

The present invention allows transient expression of the Mycl gene to be regulated to cause proliferation of pancreatic islet-like cells and differentiation into insulin-producing cells. The invention is thus characterized by transient expression of the Mycl gene, but when the Mycl gene is transiently expressed, the relative number of cells in which the Mycl gene has been introduced decreases as the pancreatic islet-like cells undergo cell division, and therefore expression level of the Mycl gene can naturally decrease or stop as time progresses. In other words, according to this embodiment the object of the invention is achieved by "on" regulation of Mycl gene expression. According to another embodiment, Mycl gene expression can be forcibly regulated "off" (also referred to hereunder as "on/off" regulation).

Using reverse tetracycline in this manner allows on/off regulation of expression of the Mycl gene that has been introduced into the host cells. In other words, intracellular expression of the Mycl gene can continue in the presence of reverse tetracycline, causing proliferation of pancreatic islet-like cells, while removing reverse tetracycline can halt proliferation of pancreatic islet-like cells and induce their differentiation to insulin-producing cells. According to the invention, the transient expression ("on" state) of the Mycl gene introduced into the pancreatic islet-like cells is preferably for a period of from at least 2 days to a maximum of 100 days, such as 90 days, 80 days, 70 days or 60 days, after the start of cell culturing. The pancreatic islet-like cells into which the Mycl gene has been introduced preferably proliferate during this period.

An alternate means of on/off regulation of Mycl gene expression is an "photoregulated viral vector" (Tahara, M., et al., PNAS, vol. 116, 11587-11589, 2019) that allows gene expression of a viral vector and cell proliferation to be precisely switched on and off by photoirradiation. Such a vector has a gene coding for an optical switch protein known as a magnet introduced into a viral vector, with expression of the target gene incorporated into the same vector being controlled using blue light.

Other alternative examples for on/off regulation of Mycl gene expression include an episomal vector (Okita K., et al., Nat Methods 2011 May 8(5):409-412), Sendai virus or an RNA vector (Warren L., et al., Cell Stem Cell 2010 Nov 7(5):618-630). There is no limitation to these, and methods using reprogramming (establishment) of iPS cells (i.e. methods of temporarily inducing genes) may also be used.

The object of the invention can also be achieved by causing forced expression of the Mycl gene present in cells instead of introducing an isolated exogenous Mycl gene. The means for causing expression of an endogenous Mycl gene is not limited, and it may be a method of replacing a wild type promoter or enhancer with a strong promoter or enhancer for inducing operable expression of the Mycl gene, and forcibly causing gene expression. Examples of promoters to be used for such replacement include the strong promoters: cytomegalovirus (CMV) promoter, and inducible promoters that function in the presence of inducers. Examples of enhancers to be used for the replacement include SV40 enhancer, herpes B virus enhancer, cytomegalovirus enhancer and α-fetoprotein enhancer. According to another embodiment, a molecule may be used that has promoter- and/or enhancer-activating demethylating enzyme, histone-modifying enzyme or transcription activator, such as VP64, p65 or Rta, added to the genome-recognition sequence of CRISPR TypeII (including CRISPR-dCas9), or CRISPR-type I, TALEN or ZFN. Throughout the present specification, the promoter, enhancer and other activating enzymes and factors, or nucleic acid protein complexes or low molecular compounds, may be referred to as "activators" for activation of the Mycl gene.

### (6) Introduction of Mycl gene product

Introduction of Mycl gene product into cells may be carried out by a common method for introducing exogenous genes or proteins into cells. Such methods include, but are not limited to, methods using transfection reagents, methods using viruses, electroporation methods, particle gun methods, sonoporation methods, liposome fusion methods and transfer methods in which pores are formed in cell membranes using a micromanipulator or laser light irradiation.

### (7) Creation of chimeric mammals

The method of introducing ES cells into a mammal to create a chimeric mammal may be a method that is well known to those skilled in the art. First, any medium known to those skilled in the art may be used for culturing of ES cells into which the Mycl gene has been introduced. For example, when the ES cells are to be cultured on feeder cells, the feeder cells used may be MEF (mouse embryonic fibroblasts), and the ES cells may be cultured on the feeder cells using ES cell medium (for example, knockout DMEM (GIBCO) containing 15% FBS, 50 U/mL penicillin/streptomycin, L-glutamine and non-essential amino acids, with addition of 2-mercaptoethanol (2ME, GIBCO) and LIF (SIGMA).

The ES cells are then introduced into a mammal to create a knockout animal (Mycl gene knock-in animal). Mice were used here as an example of mammals, and methods of creating knock-in mice are well known to those skilled in the art. Specifically, the ES cells may be injected into a mouse (such as C57BL/6) blastocyst, which is then transplanted into the uterus of a pseudopregnant female mouse (such as ICR) to create a chimeric mouse. The chimeric mouse may then be cross-bred with a normal mouse such as C57BL/6) to create a hetero mutant mouse with hetero knock-in of the Mycl gene. Cross-breeding of hetero mutant mice can produce a homozygous mouse with homo knock-in of the Mycl gene. Creation of the knock-in mice may be as for ECAT3 knock-in mice (Tokuzawa, Y., et al., Molecular and Cellular Biology, 23(8):2699-2708(2003)), ECAT4 knock-in mice (Mitsui, K., et al., Cell, 113:631-642(2003)) or ECAT5 knock-in mice (Takahashi, K., K. Mitsui and S. Yamanaka, Nature, 423(6939):p541-545(2003), Japanese Unexamined Patent Publication No. 2003-265166).

Chimeric mammals can also be created using iPS cells instead of the aforementioned ES cells. For example, a blastocyst complementation method may be used to create an organ from human iPS cells in a non-human mammal. For example, Nakauchi et al. have generated human pancreas derived from human iPS (Nakauchi, H., et al., PNAS, Vol. 110, No. 1, 4557-4562(2013)) in the bodies of pancreas-lacking cloned pigs.

### 2. Regulation of proliferation and differentiation of pancreatic islet-like cells

According to the invention it is possible to control proliferation and differentiation of pancreatic islet-like cells created as described above, both *in vitro* and *in vivo.* The method of inducing the Mycl gene can be selected according to the method used for introduction of the gene into cells. For example, when the TetOP-Mycl-ires-mCherry vector has been used as the gene transfer vector for introduction of Mycl gene into cells, it binds with reverse tetracycline regulatory transactivator expressed by host cells in a manner dependent on a reverse tetracycline such as doxycycline (Dox), and the Mycl gene linked downstream from it is expressed, to allow induction of proliferation of pancreatic islet-like cells. The concentration of Dox added to the cell culture system may be adjusted as appropriate. It may be 1 to 100 mg/mL, for example. After pancreatic islet-like cells have proliferated by addition of Dox, the proliferation can be stopped by replacement with Dox-free medium, for example, to induce cell differentiation into insulin-producing cells.

Addition of Dox-containing water to a chimeric non-human mammal *in vivo* can induce proliferation of pancreatic islet-like cells in the pancreas. The Dox concentration when water has been added may be 1 to 100 mg/mL and preferably 2.0 mg/mL, for example. When a chimeric non-mouse is used, since the pancreas grows with increasing age up to 8 weeks after birth, Dox may be administered during the period when growth has ceased, such as after 8 weeks from birth, but there is no limitation to the number of weeks so long as proliferation can be induced.

### 3. Method for producing pancreatic islet cells or progenitor cells with insulin-producing ability

The invention provides a method for producing pancreatic islet cells or their progenitor cells which have insulin-producing ability. The production method is not restricted but includes using primary islet cells, cultured pancreatic islet cells or stem cell-derived pancreatic islet cells as the source, introducing the Mycl gene into the pancreatic islet cells, and causing proliferation of the cells by forced expression of the Mycl gene, and then stopping expression of the gene to obtain pancreatic islet cells or progenitor cells with insulin-producing ability. As explained above, forced expression and stopping of the Mycl gene may utilize alternative means for on/off regulation of the gene expression (such as using a photoregulated viral vector), or a doxycycline-sensitive reverse tetracycline regulatory transactivator. The term "pancreatic islet progenitor cells" or "pancreatic islet precursor cells" used herein refers to cells (or a cell group) on the pathway of differentiation to pancreatic islet cells with insulin production ability after expression of the Mycl gene has been stopped, and they can be identified using one or more markers selected from the group consisting of PDX1 positivity, PTFla positivity, NKX6.1 positivity, Fev positivity, Pax4 positivity, and the Cck gene.

### 4. Use as a drug

According to the invention it is possible to control expression of the introduced Mycl gene to cause proliferation of pancreatic islet-like cells and promote insulin production. According to one aspect, therefore, the invention provides a pancreatic islet cell proliferation promoting agent, pancreatic islet function improver or insulin production promoting agent that includes the Mycl gene or its gene product as an active ingredient; a pharmaceutical composition comprising the active ingredient and other pharmaceutically acceptable components (such as carriers, excipients, disintegrators, buffering agents, emulsifying agents, suspending agents, soothing agents, stabilizers, preservatives, antiseptic agents or physiological saline); a method for preventing and/or treating a diabetes patient using the pancreatic islet cell proliferation promoting agent, pancreatic islet function improver, insulin production promoting agent or pharmaceutical composition; and the use of the Mycl gene for production of the pancreatic islet cell proliferation promoting agent, pancreatic islet function improver, insulin production promoting agent or pharmaceutical composition. According to another aspect, the invention provides a method of administering the Mycl gene or its gene product for the purpose of preventing and/or treating diabetes *(in vivo* method); and a method of grafting pancreatic islet-like cells, pancreatic islet precursor cell-like cells or insulin-producing cells (hereunder also collectively referred to simply as "pancreatic islet-like cells") into which the Mycl gene or its gene product has been introduced *in vitro,* or any desired combinations of the foregoing (*ex-vivo* method).

The *ex-vivo* method of the invention may be intended for a different aspect of the invention, depending on the cells used. Examples include (i) the aspect of heterologous *in vitro* or autologous *in vitro* production of pancreatic islet-like cells obtained by gene transfer into cells derived from adult pancreatic islets (the *ex-vivo* method including both in the narrow sense); (ii) the aspect of heterologous *in vitro* or autologous *in vitro* production of pancreatic islet-like cells obtained by introduction of the Mycl gene of pancreatic islet-like cells obtained by inducing differentiation, i.e. pancreatic islet cells derived from stem cells (iPS cells, ES cells, somatic stem cells, etc.) (the *ex-vivo* method in the wide sense); (iii) the aspect of proliferating pancreatic islet precursor cell-like cells obtained by functional expression of the Mycl gene in (i) and (ii) above; and (iv) the aspect of insulin-producing cells or pancreatic islet cells (without the Mycl gene) produced by differentiation from (i) to (iii) above.

According to one aspect of the invention, the cells may be either autologous or heterologous with respect to the patient and administered by a publicly known method, and specifically by using the pancreatic islets to be grafted in encapsulated form, by the method described in Nature Medicine volume 22, pp.306-311(2016)., doi:10.1038/nm.4030, Nature Biomedical Engineering volume 2, pp.810-821(2018)., DOI:10.1038/s41551-018-0275-1, EBioMedicine 12 (2016) 255-262., DOI:https://doi.org/10.1016/j.ebiom.2016.08.034. Encapsulation of the pancreatic islets to be grafted makes it possible to avoid the use of immunosuppressive agents or to reduce the dosage of immunosuppressive agents.

According to one aspect, isolated pancreatic islets may be gene-edited as appropriate. There are no particular restrictions on the gene editing, and specifically, preferred characteristics may be added during treatment, such as by repair of a mutant gene in the pancreatic islet cells by genome editing, modification of immunoreaction-inducing molecules including surface antigens such as HLA or GAD protein, or induction of immunological tolerance by deletion of beta-2 microglobulin or the RFX5, RFXANK, RFXAP or CIITA genes. The method of gene editing is not particularly restricted, and for example, it may employ a transposon vector such as piggyBAC, for expression of CRISPR-Cas9, TALEN, ZFN, CRISPR-Cas3, CRISPR-TypeI-D or their modified forms, or functional molecules.

### (1) Indications

The diseases to be targeted by the Mycl gene or its gene product based on the aspects described above are diseases in which insulin is insufficiently functioning in the body (typically insulin resistance or decreased insulin secretion). According to the invention, insulin production can be promoted by the Mycl gene or its gene product, thereby producing an effect of lowering blood sugar in a diabetes patient, for example. The disease to be targeted will typically be diabetes, but more specifically it is a disease, disorder or symptoms associated with severe hypoglycemia, type I diabetes (including latent progressive type-1 diabetes or type 1.5 diabetes), type II diabetes, impaired glucose tolerance, hyperglycemia, heterolipidemia, obesity or metabolic syndrome, or another specific mechanism or disease, examples of which include genetic abnormalities associated with pancreatic β cell function, genetic abnormalities associated with insulin function transfer mechanisms, or other diseases or conditions including pancreatic secretion diseases, endocrine diseases, liver disease, drug agent or chemical substance-induced disease, infectious disease or rare immune mechanism-related conditions, as well as pregnant diabetes. Diabetes complications resulting from diabetes (such as diabetic retinopathy and diabetic neuropathy) may also be target diseases. Insulin hyposecretion resulting from pancreas removal or partial pancreas excision performed due to pancreatitis or pancreatic cancer, may also be a target disease.

There is no particular restriction on the type of diabetes to be treated by the method described herein, but the method can provide treatment wherein physiological insulin is secreted in a manner dependent on blood glucose level, and is unlikely to cause hypoglycemia. When treating severe hypoglycemia, for example, since it is known to those skilled in the art that donor pancreatic islets exhibit a marked effect against severe hypoglycemia, a method described in the published literature may be used as one aspect. The method described in the published literature may be, but is not restricted to, the specific methods described in Diabetes Care 2016 Jul; 39(7): 1230-1240., DOI: 10.2337/dc15-1988, The New England Journal of Medicine. 343 (4):230-238., DOI: 10.1056/NEJM20,0007273430401, and The New England Journal of Medicine. 355 (13):1318-1330., DOI:10.1056/NEJMoa061267. Alternatively, instead of cell grafting as described in the published literature, a method of administering the Mycl gene or its gene product to a target *(in vivo* method) may be selected, which method can increase pancreatic islet cells, pancreatic islet-like cells, pancreatic islet precursor cell-like cells or insulin-producing cells to the same level, and preferably a higher level, than the amount of grafted pancreatic islet cells as described in the literature.

For use in treatment of type I diabetes, one aspect for treatment of type I diabetes may be, but is not restricted to, optionally adjusting the number of pancreatic islet cells, pancreatic islet-like cells, pancreatic islet precursor cell-like cells or insulin-producing cells according to the insulin level, blood glucose level and/or C-peptide level, either constantly or during fasting, after glycemic load and/or after glucagon stimulation in a type I diabetes patient, and determining the dose of the agent for *in vivo* treatment by the method described herein. Considering the high risk of hypoglycemia for insulin-depleted type I diabetes patients, and the desirability of treatment using the method described herein, a type I diabetes patient with a C-peptide level of 0.5 ng/mL or lower, preferably 0.2 ng/mL or lower and more preferably 0.1 ng/mL or lower may be treated during fasting and/or during glucagon stimulation. A specific dose for the agent may be adjusted in light of transplant amounts of donor pancreatic islets that are commonly grafted to treat severe hypoglycemia. Specifically, the pancreatic islet cells, pancreatic islet-like cells, pancreatic islet precursor cell-like cells or insulin-producing cells may be in an equivalent of 500 IEQ/kg or greater, preferably 1000 IEQ/kg or greater, more preferably 2000 IEQ/kg or greater and even more preferably 5000 IEQ/kg or greater. Alternatively, a method of administering the Mycl gene or its gene product capable of producing an increase in the same amount of pancreatic islet-like cells, pancreatic islet precursor cell-like cells or insulin-producing cells *(in vivo* method) may be selected.

For treatment of type II diabetes, one aspect may be, but is not limited to, use as a treatment agent for insulin-dependent type II diabetes having an insufficient amount of insulin secreted in the body. Specifically, optionally adjusting the number of pancreatic islet cells, pancreatic islet-like cells, pancreatic islet precursor cell-like cells or insulin-producing cells according to the insulin level, blood glucose level and/or C-peptide level, either constantly or during fasting, after glycemic load and/or after glucagon stimulation in a type II diabetes patient, and determining the dose of the agent for *in vivo* treatment by the method described herein. Considering that a desirable effect can be obtained for pathologies with insufficient insulin, a type II diabetes patient with a C-peptide level of 0.5 ng/mL or lower, preferably 0.2 ng/mL or lower and more preferably 0.1 ng/mL or lower may be treated during fasting and/or during glucagon stimulation. A specific dose for the agent may be adjusted in light of transplant amounts of donor pancreatic islets that are commonly grafted to treat severe hypoglycemia. Specifically, the pancreatic islet cells, pancreatic islet-like cells, pancreatic islet precursor cell-like cells or insulin-producing cells may be in an equivalent of 500 IEQ/kg or greater, preferably 1000 IEQ/kg or greater, more preferably 2000 IEQ/kg or greater and even more preferably 5000 IEQ/kg or greater. Alternatively, a method of administering the Mycl gene or its gene product capable of producing the same amount of pancreatic islet-like cells, pancreatic islet precursor cell-like cells or insulin-producing cells *(in vivo* method) may be selected.

For use as a treatment agent for latent progressive type I diabetes, it may be, but is not limited to, adjusting the amount of agent according to the amount of blood glucose level and/or C-peptide, in the same manner as for type I diabetes and or type II diabetes. In this case, the pancreatic islet cells, pancreatic islet-like cells, pancreatic islet precursor cell-like cells or insulin-producing cells caused to proliferate by the method described herein are preferably derived from the patient. For example, as one preferred aspect for treatment of latent progressive type I diabetes, if the autoantibody for pancreatic islet cells or the HLA type in blood can be examined beforehand and autologous pancreatic islet cells can be increased by the method described herein either before or after an insulin-dependent condition and/or insulin-depleted condition has been established, then treatment will not require an immunosuppressive agent, and progression to an insulin-dependent condition and/or an insulin-depleted condition can be prevented, or the insulin-dependent condition and/or insulin-depleted condition can be treated. In order to identify whether the condition is latent progressive type I diabetes, if an autoantibody for pancreatic islet cells or the HLA type in blood is contributing to latent progressive type I diabetes, then a publicly known method may be used, such as, though not limited to, indicating whether or not the patient is positive for one or more antibodies from among pancreatic islet-related autoantibody such as pancreatic islet cell antibody (ICA), GAD antibody, insulin autoantibody (IAA) or IA-2 antibody. It may also be confirmed, by a known method, whether or not HLA associated with latent progressive type I diabetes, such as HLA-DR4-DQA1∗0301-B1∗0401, is retained.

Considering that a therapeutic effect is exhibited by donor pancreatic islets, treatment of severe hypoglycemia is preferably by an *ex-vivo* method, but pancreatic islet cells may also be increased in the body *(in vivo)* by the method described herein. Since the increased pancreatic islet cells have the same effect as an *ex-vivo* method, a treatment method by increasing pancreatic islets *in vivo* may likewise be suitably selected as a treatment method for severe hypoglycemia, similar to an *ex-vivo* method. For treatment of type I diabetes, type II diabetes and latent progressive type I diabetes as well, a method of increasing pancreatic islet cells in the body *(in vivo)* may be selected, similar to an *ex-vivo* method, in consideration of the gender, age, body weight, affected state of the patient, and state of the cells used. A method of increasing pancreatic islet cells in the body *(in vivo)* may also be combined with an *ex-vivo* method.

According to the invention it is possible to prevent and/or treat such diseases. The term "prevent", as used herein, means stopping or delaying onset/development or lowering the risk of onset/development of the disease or its symptoms.

The term "treatment" includes mitigation (alleviation) of characteristic symptoms or accessory symptoms of a target disease, and arrest or retardation of aggravation of symptoms, and treatment also includes improvement in the disease.

### (2) Insulin production promoting agent and pharmaceutical composition

The Mycl gene or its gene product of the invention can be provided as a pancreatic islet cell proliferation promoting agent, pancreatic islet function improver, insulin production promoting agent or pharmaceutical composition for prevention and treatment of the aforementioned target disease. One aspect of the pancreatic islet cell proliferation promoting agent is an agent that causes proliferation of pancreatic islet cells of any one type, and preferably any two or more types, from among α cells, β cells, δ cells, ε cells and PP cells in pancreatic islets. Another aspect of the pancreatic islet function improver is an agent that improves some or all of the functions carried out by pancreatic islets in the body when it is administered, where some of the functions of pancreatic islets include, specifically, a blood sugar regulating effect by pancreatic islet cells, a hypoglycemic effect by insulin, a glucose-producing/releasing effect by glucagon, secretion-inhibiting effects on gastrin, secretin, insulin and/or glucagon by glucagon or a gastrointestinal tract nutrient absorption-inhibiting effect by somatostatin, an appetite-regulating effect by ghrelin, and gallbladder-contraction regulating effects and appetite-regulating effects by pancreatic polypeptides. Another aspect of the insulin production promoting agent is an agent that promotes physiological insulin secretion in response to blood glucose levels, as one of the functions performed by pancreatic islets in the body. When provided as a pharmaceutical composition, it may comprise other pharmaceutically acceptable components (for example, carriers, excipients, disintegrators, buffering agents, emulsifying agents, suspending agents, soothing agents, stabilizers, preservatives, antiseptic agents, physiological saline and the like), in addition to the active ingredient which is the Mycl gene or its gene product used according to the aspects described above. If necessary, the composition may also include an activator for activation of the Mycl gene.

Pancreatic islet cells derived from the body in which the Mycl gene or its gene product has been introduced may be pancreatic islet cells of a healthy individual, or pancreatic islet cells from a type I diabetes patient with loss of some or most β cells, or an end stage of type II diabetes patient. The pancreatic islet cells may be either autologous or heterologous with respect to the recipient.

Without being limitative, the cell preparation and pharmaceutical composition of the invention may be obtained by suspending the pancreatic islet-like cells in physiological saline or an appropriate buffer solution (for example, phosphate-buffered saline). The number of cells necessary for treatment can be produced by causing forced expression of the Mycl gene for appropriate proliferation of the cells.

For use of the pancreatic islet cells, pancreatic islet-like cells, pancreatic islet precursor cell-like cells or insulin-producing cells in a cell preparation or pharmaceutical composition, dimethyl sulfoxide (DMSO) or serum albumin may be added to the cell preparation or pharmaceutical composition to protect the cells, and an antibiotic or the like may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like) may be added to the cell preparation or pharmaceutical composition. A person skilled in the art may add such factors and chemical agents to the cell preparation and pharmaceutical composition in appropriate concentrations.

The number of pancreatic islet cells, pancreatic islet-like cells, pancreatic islet precursor cell-like cells or insulin-producing cells in the cell preparation and pharmaceutical composition to be prepared may be appropriately adjusted so as to obtain the desired effect for prevention and/or treatment of diabetes or its associated condition (for example, lowered blood glucose level), in consideration of the target gender, age and body weight, the state of the affected area, and the state of the cells to be used.

The insulin production-promoting agent and pharmaceutical composition of the invention may be administered to any of a variety of targets, including mammals such as primates, humans, dogs, cats, cows, horses, pigs or sheep, and preferably humans. The route of administration to the target is not restricted, and administration may be at any location that can produce a response to glucose in the body, by parenteral administration, such as injection or infusion, for example. Specifically, grafting or administration may be in the pancreas, under the renal capsule, preferably subcutaneous or intraperitoneal, more preferably intravascular or intravenous, and even more preferably intraportal.

The method of activating the administered Mycl gene in the body is not restricted, and a system that regulates "on" expression, or regulates "on and/or off' expression, of the Mycl gene may be used. For example, the "photoregulated viral vector" (Tahara, M., et al., PNAS, vol. 116, 11587-11589, 2019) mentioned above may be used.

For targeting of the Mycl gene into the pancreas, gene transfer may be carried out with a marker that is specifically expressed in pancreatic islet cells (for example, PDX1, C-peptide, insulin, MafA, Mnx1, Pax4, Pax6, NeruroD1, Isl1, Nkx2.2, Ngn3, HNF1a, Foxa2, Nkx6.1, glucagon, Arx, MafB, RFX6, IRX1, IRX2 or somatostatin) as the target. When a method of increasing pancreatic islet cells in the body *(in vivo)* is selected, the route of administration to the target is not restricted and may be, specifically, into the pancreas, subcutaneous or intraperitoneal, preferably intravascular or intravenous, and even more preferably into the celiac artery or into the pancreatic ducts.

### (3) Treatment method

According to the invention, the Mycl gene or its gene product, and pancreatic islet-like cells, or a combination of the foregoing, are used to provide a method of preventing and/or treating diabetes or its associated condition. Moreover, in the treatment method of the invention it is possible to administer an activator that can activate the Mycl gene, either before or simultaneously with administration of the insulin production promoting agent or pharmaceutical composition.

### (4) Kit

The present invention also provides a kit to be used for preventing and/or treating diabetes or its associated condition, which includes an insulin production promoting agent or pharmaceutical composition. Such a kit may also include an instruction manual for administration or grafting of the insulin production promoting agent or pharmaceutical composition. The kit may further include an activator for activation of the Mycl gene.

### EXAMPLES

While the present invention will be described in further detail by the following Examples, the present invention is not limited by these Examples.

### Method

### (i) Establishment of ES cells capable of inducing Myc, Mycn, and Mycl expression in a Dox-dependent manner

Myc, Mycn, and Mycl cDNAs were cloned from ES cell-derived cDNA, and the cloned fragments were inserted into a pCR8-GW-TOPO vector (Invitrogen). A col1a1-TetOP-Mycl-ires-mCherry vector (hereinafter referred to as a "targeting vector") produced by LR reaction between a pCR8-Mycl-TOPO vector with each Myc gene inserted and a TetOP-AtR1-cdB-AttR2-ires-mCherry vector was inserted at the Col1a1 locus of KH2-ES cells by using a flip-in recombination system (Beard, et al., 2006). For the flip-in recombination, a cell suspension suspended in high glucose DMEM (Nacalai Tesque, Inc.) medium containing 50 µg of each Myc gene inserted targeting vector and 25 µg of pFlapase vector and 25 mM HEPES buffer (Gibco Co. Ltd.) was electroporated (Voltage: 550 V, Capacitance: 25 µF, Resistance: ∞, Cuvette: 2 pulses at least 4 mm) into KH2-ES cells using a Gene Purser Xcell electroporation system (BIO-RAD). Twenty-four hours after electroporation, hygromycin B (Roche, Ltd.) was selected at 150 µg/mL to pick colonies formed and establish ES cell lines capable of inducing expression of each Myc gene in a Dox-dependent manner.

### (ii) Cell culturing method

Feeder cells (MEF; mouse fetal fibroblasts) were cultured using DMEM (Nacalai Tesque, Inc.) medium containing 10% FBS (Gibco Co. Ltd.), 50 U/mL Penicillin-Streptomycin (P/S; Nacalai Tesque, Inc.), L-glutamine (Gibco Co. Ltd.) and NEAA (Nacalai Tesque, Inc.).

ES cells were cultured using medium in which 2-mercaptoethanol (2ME: Gibco Co. Ltd.) and LIF (Sigma-Aldrich, Co. LLC) were added to knock out-DMEM (Gibco Co. Ltd.) with 15% FBS, 50 U/mL P/S; L-glutamine, and NEAA, on gelatin-coated (Sigma-Aldrich, Co. LLC) dishes seeded with feeder cells. During the passage of ES cells, the cells were treated with 0.25% trypsin/1 mM EDTA (Gibco Co. Ltd.) at 37°C for approximately 3 minutes, and approximately 1/10 volume of the cell suspension was seeded into a new dish.

### (iii) Preparation of mice capable of inducing Mycl expression in vivo

The ES cells capable of inducing Mycl expression in a doxycycline (Sigma-Aldrich, Co. LLC, hereinafter sometimes referred to as "Dox")-dependent manner were injected into a mouse blastocyst (ICR, E3.5), and a chimeric mouse having cells capable of inducing Mycl expression in a Dox-dependent manner was prepared by transplanting the blastocyst into the uterus of a pseudopregnant day 2 mouse (Slc: ICR, Shimizu laboratory material).

### (iv) Administration of doxycycline

Eight-week-old mouse was used, and a solution containing 2.0 mg/mL of Dox was administered in drinking water. For cultured cells, Dox was added to the medium so that the final concentration was 2.0 µg/mL.

### (v) Preparation of pathological specimens from each mouse organ

After dissection of the mice, each organ was shaken in 4% PFA (Wako Pure Chemical Indsutries, Ltd.) one day and transferred to 70% EtOH (diluted with 100% EtOH from Wako Pure Chemical Indsutries, Ltd.) the following day, and shaken for another day. The following day, the spin tissue processor, STR120 (Thermo SCIENTIFIC) was used to create the blocks according to the recommended protocol. The preparation of pathological specimens was entrusted to Biogate Co., Ltd.

### (vi) Immunostaining

Tissue sections were immersed in xylene (Wako Pure Chemical Indsutries, Ltd.) and then in 100% EtOH (Wako Pure Chemical Indsutries, Ltd.) for at least 30 minutes, respectively. Washed with tap water for about 10 minutes, transferred into boiled antigen-activated solution pH 9 (used in a 10-fold dilution, Nichirey Bioscience) and subjected to antigen activation treatment for 10 minutes. 200 µL of primary antibody solution diluted with blocking solution (2% BSA + 1 x PBS) at each magnification was added on tissue sections and allowed to stand for 30 minutes to 1 hour. After washing twice with 1 x PBS, two drops of secondary antibody solution were added on tissue sections and allowed to stand for 30 minutes. After washing twice with 1 x PBS, in the case of DAB staining, 150 µL of DAB solution (using a DAB substrate kit, adding and mixing one drop each of Reagents A and B to 1 mL of Elix water, then adding and mixing one drop each of Reagent C) was added to the tissue sections, and antigen-antibody reaction was carried out, and the examination was carried out under a microscope. In the case of fluorescent staining, one drop of encapsulant was added on the tissue sections, covered glass was applied, and the sections were observed under a microscope.

### <Primary antibodies (dilution factor)-Secondary antibodies used>

- Anti-mCherry antibody (Abcam Plc., 1/500) - anti-rabbit IgG antibody (Nichirey Bioscience, Inc.)
- Anti-Synaptophysin antibody (Abcam Plc., 1/500) - anti-rabbit IgG antibody (Nichirey Bioscience, Inc.)
- Anti-Chromogranin A antibody (DAKO, 1/500) - anti-rabbit IgG antibody (Nichirey Bioscience, Inc.)
- Anti-Ki67 antibody (Abcam Plc., 1/200) - anti-rabbit IgG antibody (Nichirei Bioscience, Inc.)
- Anti-Insulin antibody (DAKO) - anti-guinea pig IgG antibody (BIOTIUM)
- Anti-Somatostatin antibody (Santa cruz, 1/300) - anti-mouse IgG antibody (BIOTIUM)
- Anti-Glucagon antibody (Santa cruz, 1/300)-anti-mouse IgG antibody (BIOTIUM)

### (vii) RNA recovery, RNA extraction, and cDNA synthesis

For RNA recovery, cultured cells were washed with PBS (-) (Nacalai Tesque, Inc.) and the cells were lysed with 350 µL of LBP buffer. RNA extraction was performed using NucreoSpin^{®} RNA Plus (Takara Bio, Inc.) according to the recommended protocol. For cDNA synthesis, the recommended protocol was followed using the Primescript single-stranded cDNA synthesis kit (Takara Bio, Inc.).

### (viii) qRT-PCR

The recommended protocol was followed using the GoTaq qPCR master mix (Promega). Analyses were performed using the Stepone Plus system (Life Technologies). The primers and PCR reaction conditions were used as follows:

### [Table 1]

**Table 1.**

| | | | |
|---|---|---|---|
| (a) | | | Primers |

| Target gene | Fw/Rv | Length (mer) | Sequence (5'→3') |
|---|---|---|---|
| β-actin | Fw | 20 | GCCAACCGTGAAAAGATGAC (SEQ ID NO: 5) |
| | Rv | 19 | TCCGGAGTCCATCACAATG (SEQ ID NO: 6) |
| Myc | Fw | 18 | CACCAGCAGCGACTCTGA (SEQ ID NO: 7) |
| | Rv | 18 | GGGGTTTGCCTCTTCTCC (SEQ ID NO: 8) |
| Mycn | Fw | 20 | CTCCGGAGAGGATACCTTGA (SEQ ID NO: 9) |
| | Rv | 20 | TCTCTACGGTGACCACATCG (SEQ ID NO: 10) |
| Mycl | Fw | 20 | ACGGCACTCCTAGTCTGGAA (SEQ ID NO: 1 1) |
| | Rv | 21 | CCACGTCAATCTCTTCACCTT (SEQ ID NO: 12) |
| Fev | Fw | 19 | CCGTCGGAGATGGTCTTTT (SEQ ID NO: 13) |
| | Rv | 20 | CCAGGAGAAACTGCCACAAC (SEQ ID NO: 14) |
| Cck | Fw | 20 | ACTGCTAGCGCGATACATCC (SEQ ID NO: 15) |
| | Rv | 20 | TATTCTATGGCTGGGGTCCA (SEQ ID NO: 16) |
| Pax4 | Fw | 20 | GTACTTCGGGCACTTCAGGA (SEQ ID NO: 17) |
| | Rv | 20 | TTACTGTGGGGACTGGGAAG (SEQ ID NO: 18) |

### (b) PCR reaction conditions

- 95°C for 2 minutes
- 95°C (15 seconds), 60°C (1 minute) [40 cycles]
- 95°C (15 seconds), 60°C (1 minute), 95°C (15 seconds)

### (ix) Pancreatic islet isolation

Eight-week-old mice were anesthetized by intraperitoneal injection of somnopentyl (Kyoritsu Pharmaceutical Industries Co., Ltd.). After laparotomy, the opening of the duodenal common bile duct was identified, and the upper common bile duct and intestine were stopped with a Bruch's clamp. The common bile duct was nicked at the junction of the duodenum and 2 mL of M199 medium (Gibco Co. Ltd.) containing Collagenase P (Roche, Ltd) (2 mg/mL) was injected. The pancreas was then removed and digested for 11 minutes and 30 seconds in a 37°C water bath. Suspended in 25 mL of M199 medium containing 10% FBS and centrifuged (1000 rpm, 4°C, 2 minutes) twice. The supernatant was discarded and suspended in 10 mL of Histopaque (Sigma-Aldrich, Co. LLC) followed by 10 mL of M199 medium containing 10% FBS and centrifuged (1000 rpm, 4°C, 30 minutes). The supernatant was transferred to another 50 mL tube and 25 mL of M199 medium containing 10% FBS was added and centrifuged (1000 rpm, 4°C, 2 minutes).

### (ix) Pancreatic islet variance

The isolated pancreatic islets were collected in 1.5 mL silicon tubes, and 100 µL of dispersion buffer (see Table 2 below) was added. After standing at 37°C for 15 minutes, it was dispersed by pipetting.

### [Table 2]

**Table 2. Dispersion buffer**

| | |
|---|---|
| Solution 1 | 10 mL |
| Solution 2 | 10 mL |
| 0.1M EGTA | 4mL |
| Hepes | 0.1909 g |
| BSA | 0.04 g |
| H₂O | 20 mL |

(Adjusted to pH 7.4)

### [Table 3]

**Table 3.**

| | |
|---|---|
| Solution 1 | 0.64M NaCl |
| Solution 2 | 20 mM KCl |
| | 40 mM NaHCO₃ |
| | 10 mM MgCl₂-6H₂O |

### (x) Pancreatic islet culture (three-dimensional culture with gel)

After adding 20 µL of Matrigel (Corning, Inc.) containing isolated pancreatic islets to a 96-well plate (Corning, Inc.), the Matrigel was gelled by incubation at 37°C for 15 minutes. Subsequently, 140 µL of medium containing growth factors (see the table below) was added and incubated at 37°C in a 5% CO₂.

### [Table 4]

**Table 4.**

| Feeding media | | Splitting media | |
|---|---|---|---|
| A83-01 | 5 µL | DMEM-F 12 advanced | 50 mL |
| mEGF | 25 µL | HEPES Buffer | 0.5 mL |
| FGF-10 | 10 µL | Penicillin/Streptomycin | 0.5 mL |
| Gastrin I | 5 µL | Glutamax | 0.5 mL |
| mNoggin | 5 µL | | |
| N-acetylcystein | 10 µL | | |
| Nicotineamide | 50 µL | | |
| R-Spondin | 50 µL | | |
| B27 supplyment | 100 µL | | |
| Splitting media | 5 mL | | |

### (xi) Pancreatic islet culture (suspension culture)

Two mL of medium was added to 6-well plates and cell culture inserts (Millipore, co.) were suspended on the medium. Isolated pancreatic islets on cell culture inserts and 20 µL of culture medium were added and cultured at 37°C in a 5% CO₂.

### (xii) Intraperitoneal glucose tolerance test (IPGTT)

The Mycl-expressing group and control group were fasted for 12-16 hours. Each mouse was then weighed and injected intraperitoneally with a D-glucose solution to form D-glucose 2 g/kg (mice). After 15, 30, 60, and 120 minutes, blood was collected from each mouse tail and blood glucose levels were measured using an anthosense table (Horiba, Ltd.).

### (xiii) Preparation of diabetic mice

Body weights of 8-12-week-old immunodeficient mice (NOD/SCID) were measured and streptozosin solutions were injected intraperitoneally into mice to a dose of 150 mg/kg streptozosin (STZ) (Sigma-Aldrich, Co. LLC). After 1 week, blood glucose levels were measured using an anthosense table (Horiba, Ltd.) and mice with blood glucose of 250 mg/dL or higher were used as diabetic mice.

### (xiv) Pancreatic islet transplantation (renal subcapsular transplantation)

Diabetic mice were anesthetized by intraperitoneal injection of somnopentyl (Kyoritsu Pharmaceutical Industries Co., Ltd.). After laparotomy, the renal capsule was dissected using an injection needle. Silicon tubes were inserted under the renal capsule and pancreatic islets were transplanted under the renal capsule using Hamilton syringe. After implantation, the silicone tube was removed and the peritoneum and coating were sutured.

### (xv) Nephrectomy

Mice implanted with pancreatic islets were anesthetized by intraperitoneal injection of somnopentyl (Kyoritsu Pharmaceutical Industries Co., Ltd.). After laparotomy, the kidneys transplanted with pancreatic islets were removed and the renal veins and arteries were ligated with ligature. After nephrectomy, the peritoneum and capsule were sutured.

### (xvi) Single-cell analysis

Reanalysis was performed using the analysis data (GSE101099; Byrnes LE et al., Nat Commun. 2018 Sep 25; 9 (1): 3922) published in the GEO database. For t-SNE analysis, Seart v2.2 and v2.3 were used (Satija R., et al., Nat Biotechnol. 2015 33: 495-502).

### Example 1: Establishment of mouse ES cells capable of inducing Mycl expression

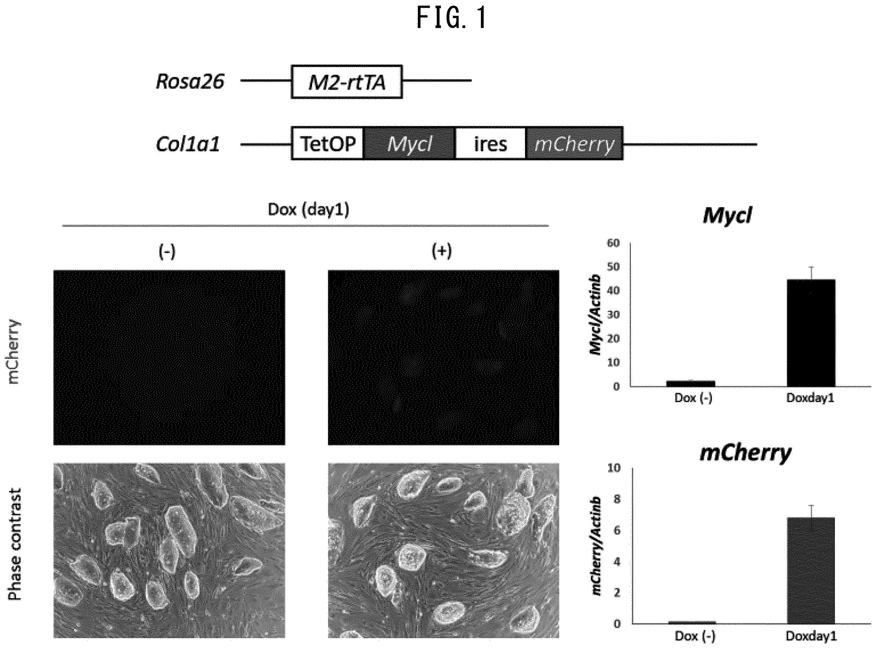

We investigated whether the ES cell lines incorporating the Mycl gene produced above could be expressed in a Dox-dependent manner. Expression of the Mycl gene can be confirmed by expression of the mCherry gene integrated downstream of the gene. The mCherry gene is a gene encoding a red fluorescent protein whose expression causes the cells to appear red. As shown in Fig. 1, comparison of gene expression before and after Dox addition revealed that Mycl gene expression was markedly increased, indicating that the mouse ES cells capable of inducing Mycl expression could be established.

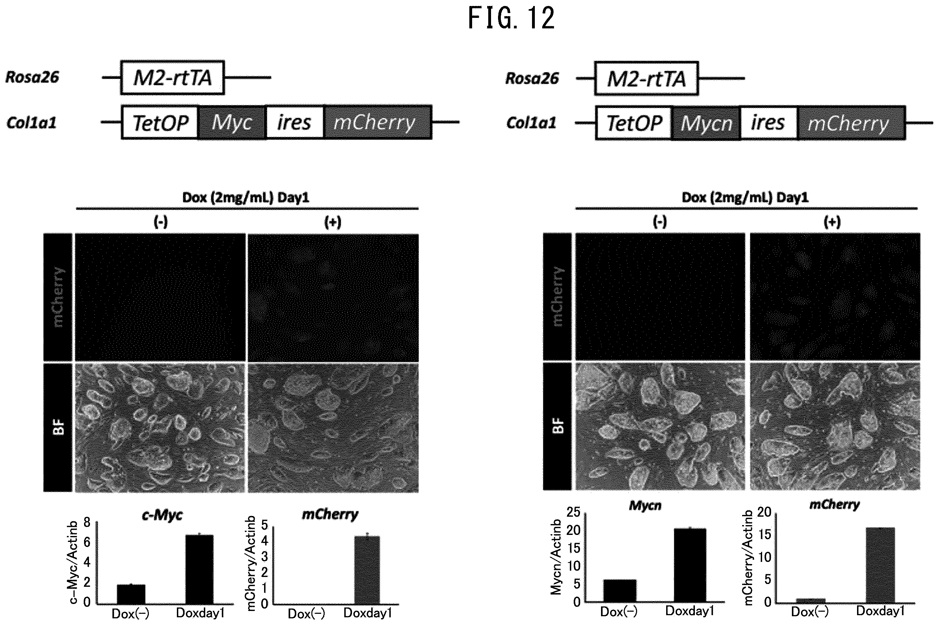

In addition, in ES cell lines incorporating each of Myc gene and Mycn gene, Dox-dependent gene expression was also induced in a similar manner. As shown in Fig. 12, expression of each gene was observed, as was the case with Mycl expression induction.

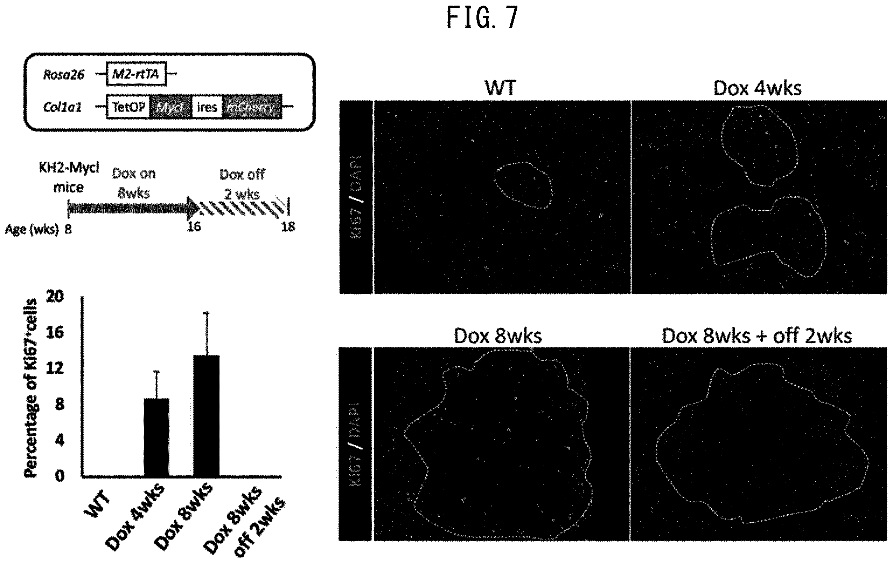

On the other hand, the expression of Ki67 protein as an indicator of cell proliferation increased with the duration of Dox treatment (Ki67-positive cells), and the number of Ki67-positive cells decreased markedly and cell proliferation stopped at 2 weeks after Dox treatment was stopped (Fig. 7). From the above results, it was found that ES cells transfected with the Mycl gene were induced to express genes by Dox and proliferated.

### Example 2: Proliferation of pancreatic islet cells by Mycl overexpression (Neuroendocrine Tumor Formation)

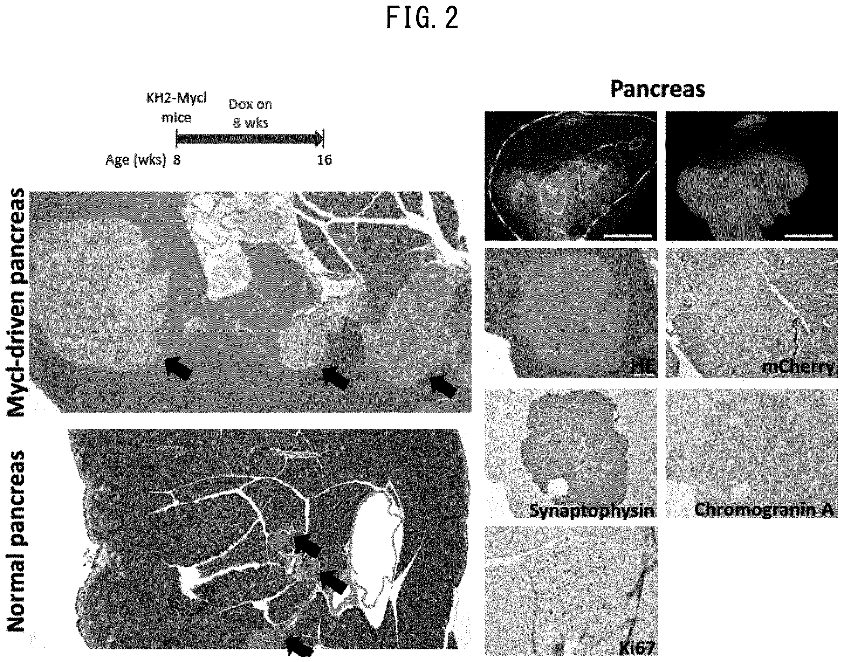

Eight-week-old chimeric mice expressing the Mycl gene produced above were treated with Dox for 8 weeks. Mice were sacrificed 8 weeks after Dox administration, the pancreas was removed, and pancreatic sections were histochemically evaluated. As indicated by the arrows in Fig. 2, pancreatic tissue overexpressing Mycl by the Dox induction shows that pancreatic islet cells proliferate and pancreatic islets are enlarged compared to normal pancreatic tissue.

### Example 3: Hyperplasia of pancreatic islet progenitor cells by Mycl overexpression

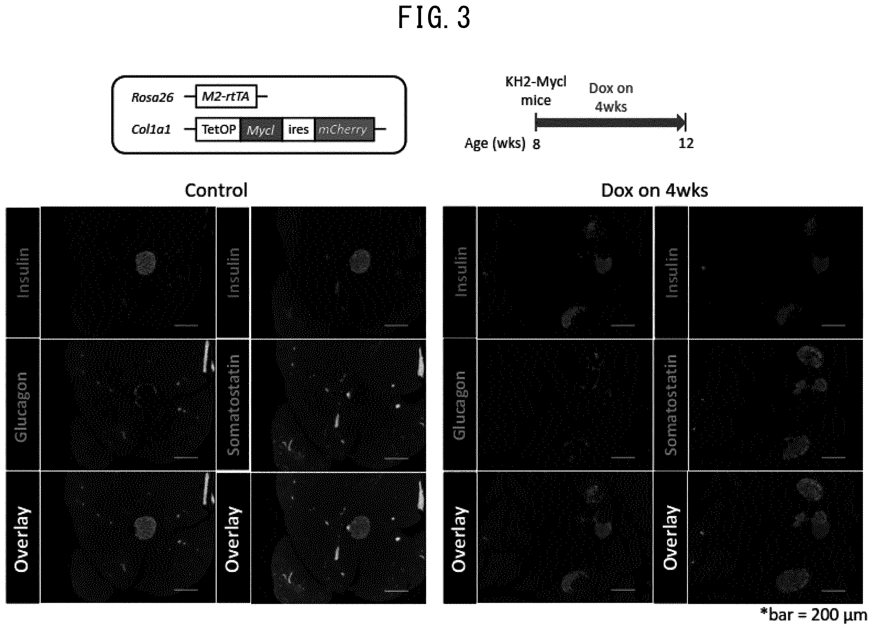

Four weeks after administration of Dox to chimeric mice, the hyperplasia of somatostatin-positive cells was investigated. As shown in Example 2, the expression of the Mycl gene in pancreatic islet cells can induce pancreatic islet cell proliferation. As shown in Figs. 3 to 6, proliferating cells exhibited gene expression similar to that of pancreatic pancreatic islet progenitor cells in the presence of the Mycl gene, as well as somatostatin expression and proliferation, whereas insulin-positive cells were reduced.

### Example 4: Hyperplasia of somatostatin-positive pancreatic islet cell-like cells by Mycl overexpression

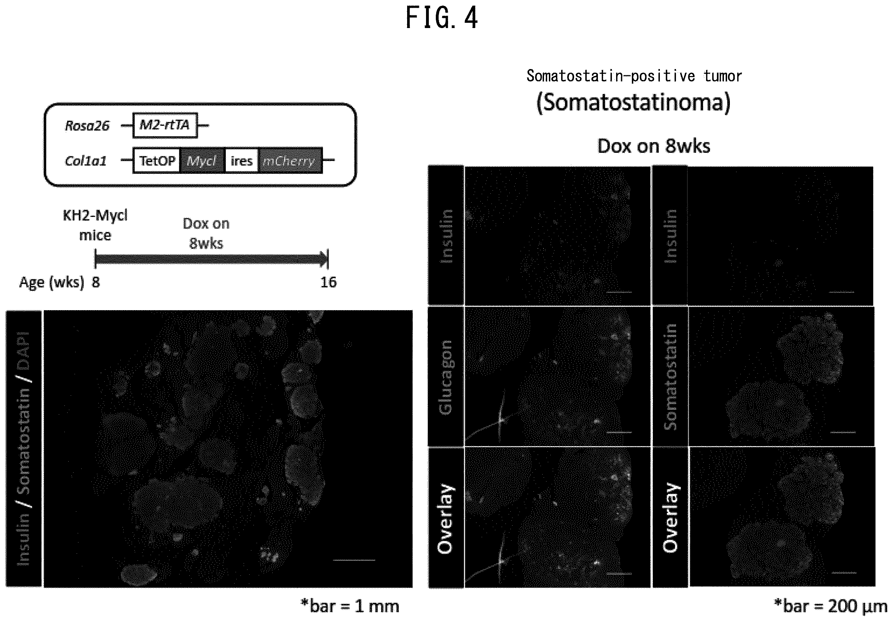

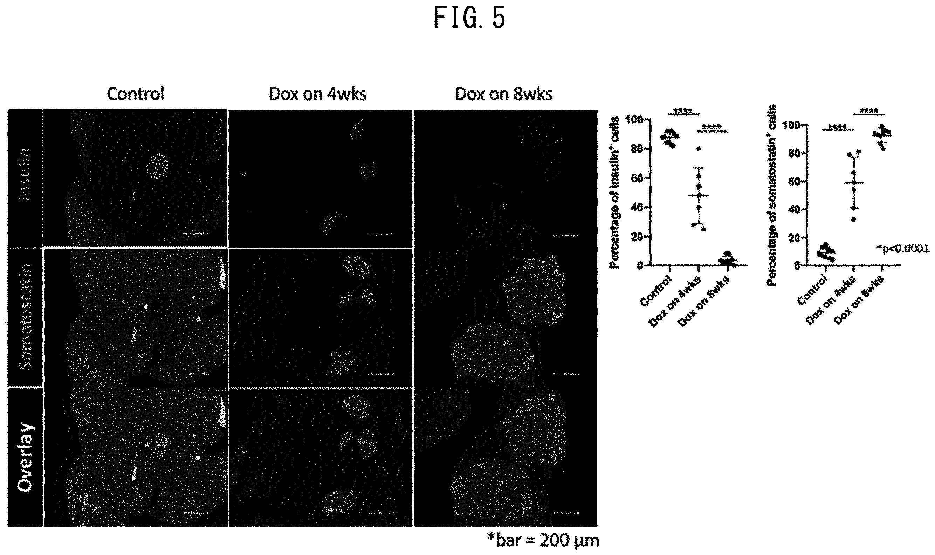

In an experimental system similar to Example 2, growth of somatostatin-positive cells and insulin-positive cells was investigated 8 weeks after Dox administration. As shown in Figs. 4 and 5, insulin-positive cells decreased and somatostatin-positive cells increased markedly.

### Example 5: Dedifferentiation to pancreatic islet progenitor-like cells by Mycl overexpression

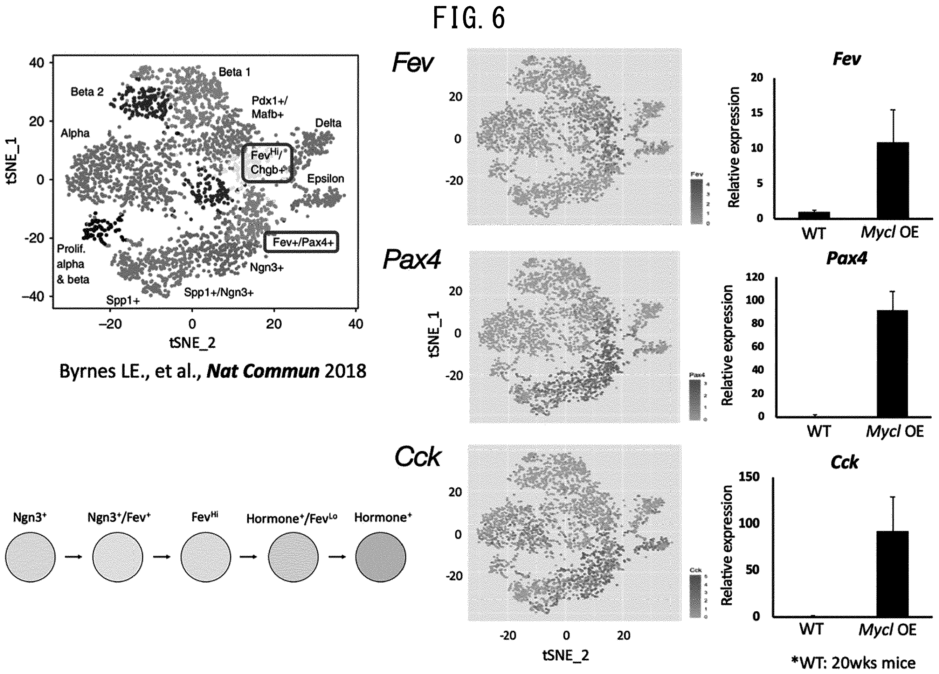

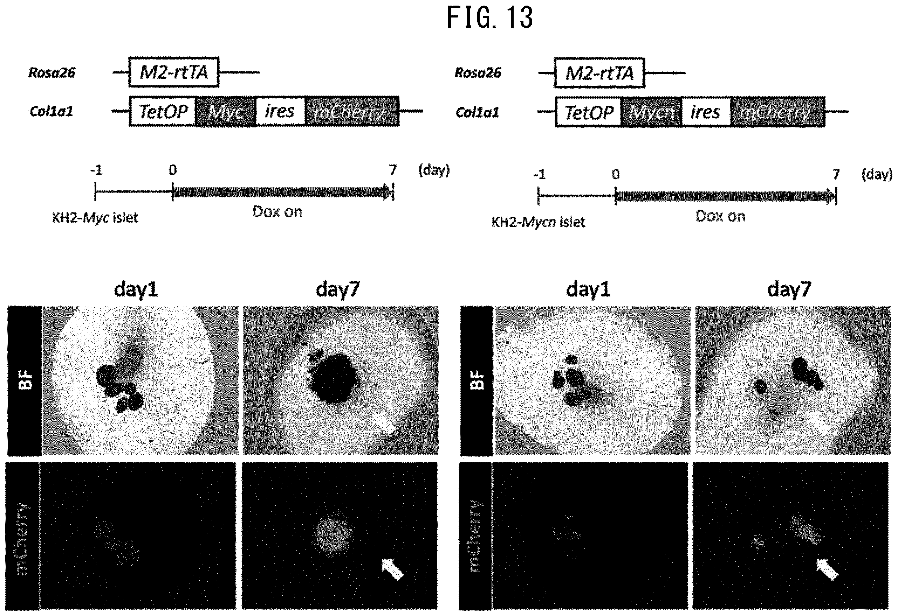

In an experimental system similar to Example 2, gene expression analysis was performed on enlarged pancreatic islets 8 weeks after Dox administration. Pancreatic islets are thought to differentiate from pancreatic islet progenitors with Ngn3 markers to pancreatic islet progenitors with Fev markers and then into each pancreatic islet cells (Fig. 6). Analysis of gene expression in pancreatic islet cells grown by Mycl overexpression revealed increased expression of the progenitor markers Fev and simultaneously expressed Pax4 and Cck (Fig. 13). These results suggest that Mycl overexpression can induce proliferatable pancreatic islet progenitor-like cells.

### Example 6: Induction of insulin-positive cells by cessation of Mycl expression

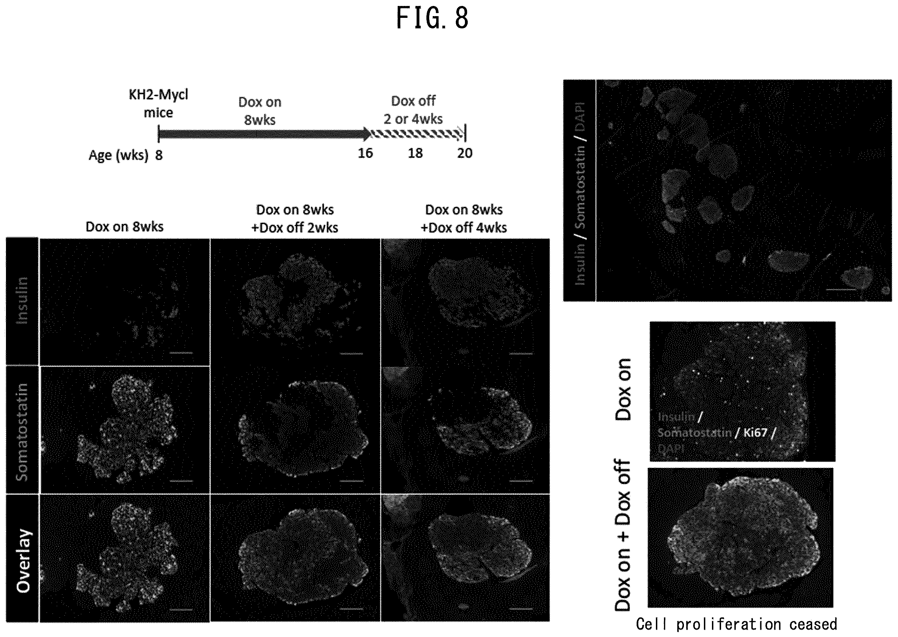

In the experimental system used in Example 2, Dox administration was stopped after 8 weeks, and the increase and decrease of insulin-positive cells and somatostatin-positive cells were observed 2 and 4 weeks later after that. As shown in Fig. 5, after 2 and 4 weeks of Dox cessation, there was an increase in the number of insulin-positive cells that decreased once, in contrast to a decrease in the number of somatostatin-positive cells that increased once (Fig. 8). These results suggest that pancreatic islet progenitor-like cells proliferated by Mycl overexpression turned into insulin-positive cells by stopping Mycl expression.

Expression of Ki67, a known target protein for cell proliferation, was observed to increase the number of proliferating cells (Ki-positive cells) as the duration of Dox administration increased, and the number of Ki-positive cells decreased markedly and cell proliferation stopped at 2 weeks after Dox administration was stopped (Fig. 7).

### Example 7: Increased glucose tolerance due to Mycl transient overexpression

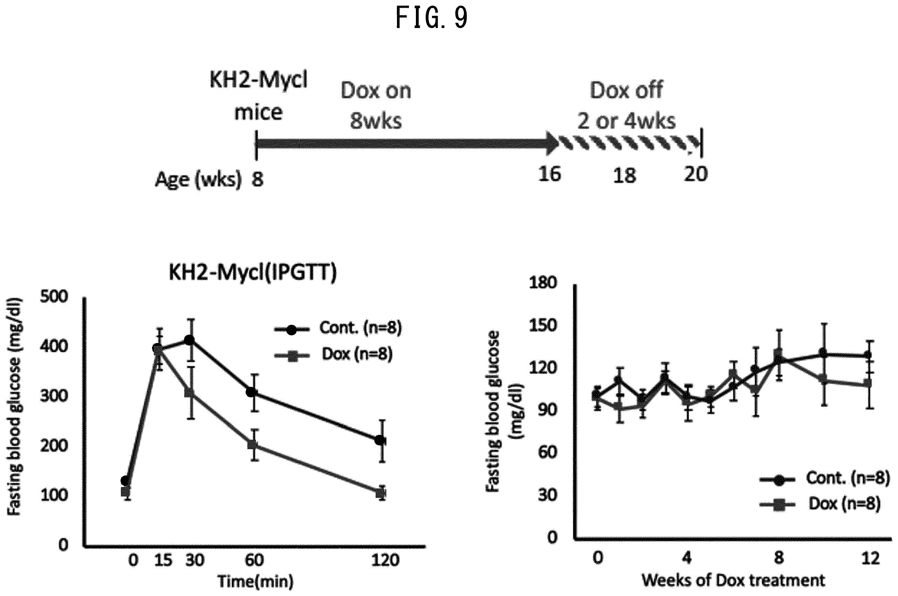

Glucose tolerance was assessed in chimeric mice when Dox treatment was stopped. As shown on the left of Fig. 9, compared to controls ("cont. "), mice that ceased Dox treatment showed increased glucose tolerance. In addition, fasting blood glucose levels were consistently maintained, suggesting that the increased pancreatic islets *in vivo* were functioning pancreatic islets (Fig. 9, right).

### Example8: Induction of proliferation of pancreatic islet cells in vitro by Mycl overexpression (1) Induction of Mycl expression in isolated pancreatic islets

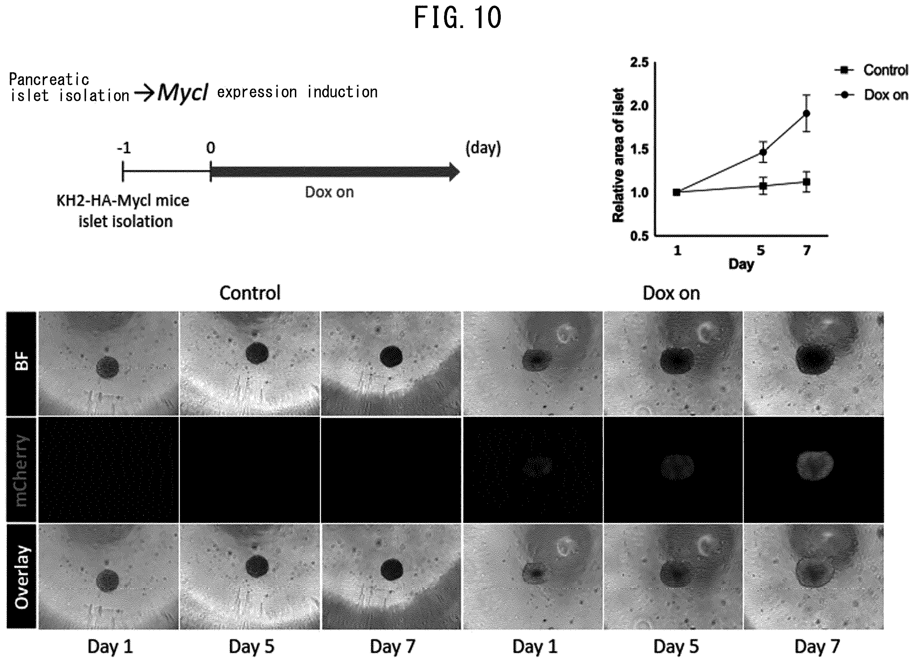

Pancreas were removed from chimeric mice and then the isolated pancreatic islets were tested for induction of Mycl expression. Fig. 10 shows the results of monitoring Mycl expression over time using the start time of dox addition as Day 0. Compared with controls, the Dox-supplemented system tended to have increased pancreatic islet size over time with the expression of the Mycl gene.

### (2) Induction of Mycl expression in cells dispersed from isolated pancreatic islets

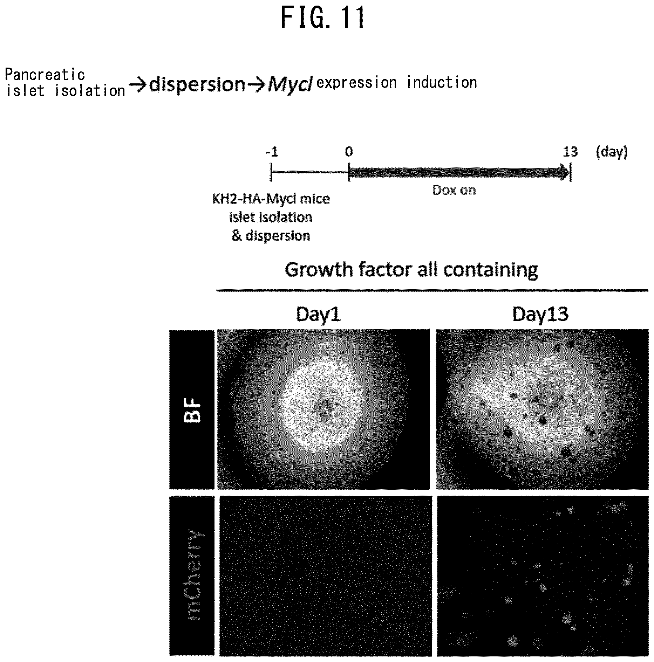

Cells dispersed from isolated pancreatic islets were tested for Mycl gene expression as in (1) above. The Mycl gene was observed in cells 14 days after addition of Dox, and it was found that Mycl gene was expressed in each cell compared to Day 1 of addition (Fig. 11).

### (3) Induction of c-Myc, Mycn expression in vitro in isolated pancreatic islets

We confirmed that the c-Myc and Mycn-incorporated ES cells produced above were able to induce expression in a doxycycline-dependent manner. The ES cells were used to generate chimeric mice. In pancreatic islet cells isolated from chimeric mice prepared, c-Myc and Mycn expression was induced by adding doxycycline *in vitro* for 1 week. The results showed that expression of c-Myc and Mycn stimulated proliferation but induced cell death, unlike Mycl (Fig. 12) (See Pelengaris S, Khan M, Evan GI., Cell 2002; 109(3):321-334).

### Example 9: Functional assessment of proliferation-induced pancreatic islet cells in vitro

*In vitro,* pancreatic islet cells induced by Mycl overexpression were transplanted into diabetic mice to investigate the functionality of proliferating pancreatic islet cells. Isolated 30 pancreatic islets were dispersed and added Dox for 1 week to induce pancreatic islet cell proliferation. These pancreatic islet cells were harvested and transplanted under the renal capsule of diabetic mice, and blood glucose was improved as needed. Two weeks later, the kidneys from which pancreatic islet cells were transplanted were removed, and diabetic mice returned to hyperglycemia. In general, more than 300 pancreatic islet transplants are required to lower blood glucose levels in diabetic mice. Pancreatic islet-like cells grown from 60 pancreatic islets were successfully used to lower blood glucose levels in diabetic mice.

### Example 10: Validation of treatment effects in Mycl expression-induced diabetes mice models

A mouse diabetes model was constructed to investigate the therapeutic effect of inducing Mycl expression. A mouse diabetes model was prepared by intraperitoneal administration of streptozocin (STZ) (toxic to pancreatic β-cells) to 8-week-old Mycl-inducible mice (KH2-Mycl) at a dose of 150 mg/kg. Mycl expression was induced by Dox for 2 weeks to 8 weeks. The expression of Mycl was stopped by stopping Dox for an additional 2 weeks. Glucose responsiveness studies (IPGTT) and histological analyses were performed at this time point.

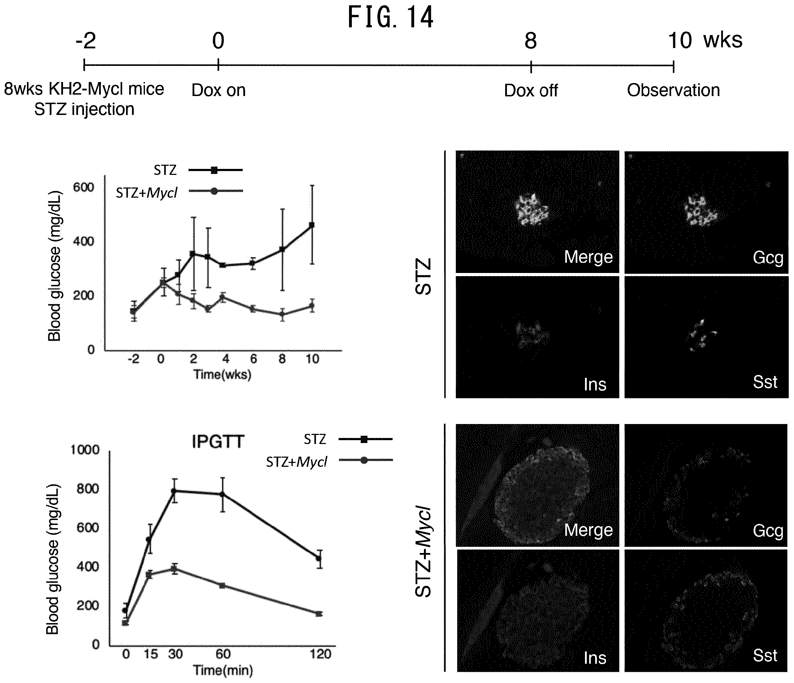

The results are shown in Fig. 14. In the control group (STZ alone), blood glucose increased after STZ administration, while in the group induced Mycl expression by Dox (STZ+Mycl), blood glucose decreased after Dox administration, and normoglycemia was observed after Dox administration (top left of Fig. 14). In addition, a glucose responsiveness test revealed a rapid decrease in blood glucose levels in the Mycl-induced group (lower left of Fig. 14).

In addition, histological analysis of the expression of insulin ("Ins"), glucagon ("Gog"), and somatostatin ("Sst") showed a reduction in the proportion of insulin-positive cells in the control group (administered STZ alone). On the other hand, in the group induced by Mycl expression (STZ+Mycl), an increase in pancreatic islet cells and an increase in the proportion of insulin-producing cells were observed. Based on these results, the induction of Mycl expression is expected to be effective in the treatment of diabetic disease.

### Example 11: Mycl does not induce abnormal growth other than pancreatic islet

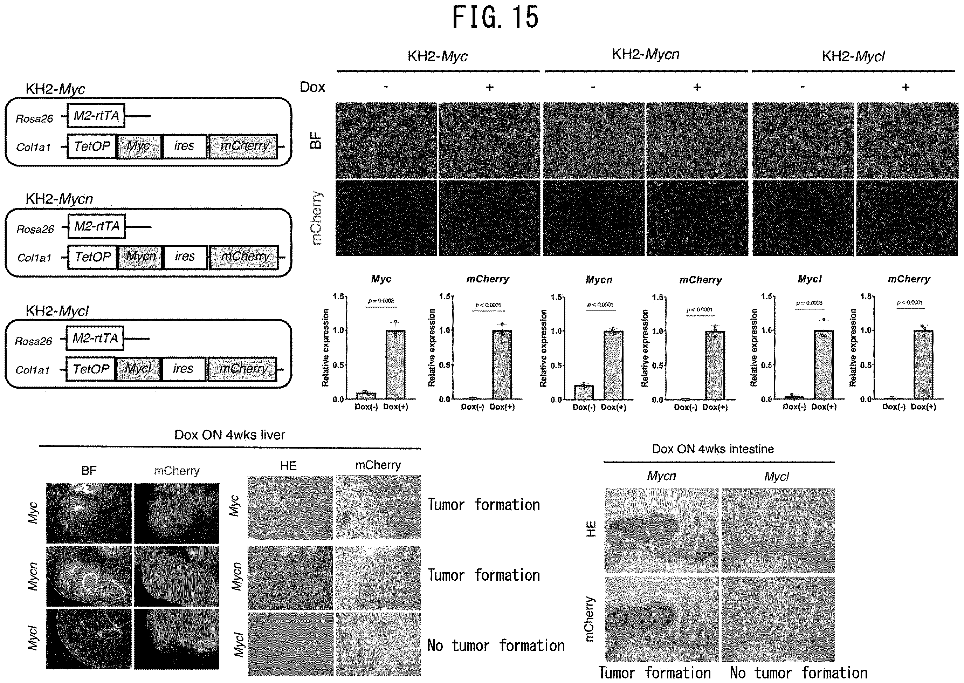

To induce the expression of the MYC family genes ("Myc", "Mycn", and "Mycl") *in vivo* in a Dox-dependent manner, we first established ES cells capable of inducing expression of these genes (top panel of Fig. 15). The ES cells were injected into blastocysts to produce chimeric mice. The MYC family genes were induced by the addition of Dox for 4 weeks from the time the chimeric mouse was 4 weeks old.

Induction of Myc expression was observed in the liver, and induction of Mycn expression was observed in the liver and intestine, whereas induction of Mycl expression was not abnormal in the liver and small intestine at least (lower part of Fig. 15). This suggests that Mycl specifically proliferates in pancreatic islets.

### Example 12: Pancreatic islet hyperplasia in elderly mice

Isolates of pancreatic islet cells were isolated from elderly mice (115 weeks old) and the Dox-dependent expression-inducible lentiviruses of the Mycl and pZsGreenDR vectors (Takara Bio, Inc., cat #632428) (control group) were infected. The zsGreenDR used is a short-lived green fluorescent protein fused with a proteolytic signal at the C-terminus of zsGreen.

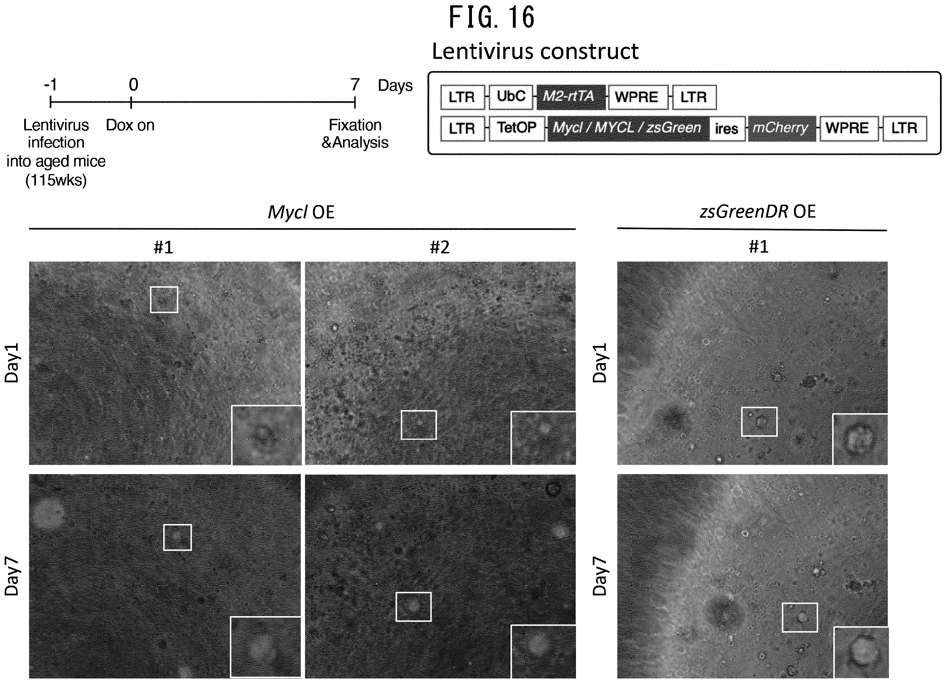

On the day following infection, the pancreatic islet cells were transferred to a three-dimensional culture using Matrigel and simultaneously administered Dox induced Mycl and zsGreenDR expression, respectively. Pancreatic islet cells expressing zsGreenDR showed no change in morphology after 7 days (right, Fig. 16). On the other hand, an increase in pancreatic islet cells was observed by inducing Mycl expression (left, Fig. 16). These results showed that Mcyl expression-induced pancreatic islet proliferation was possible in very old mice.

### Example 13: Expression of Mcyl in human pancreatic islet progenitor cells

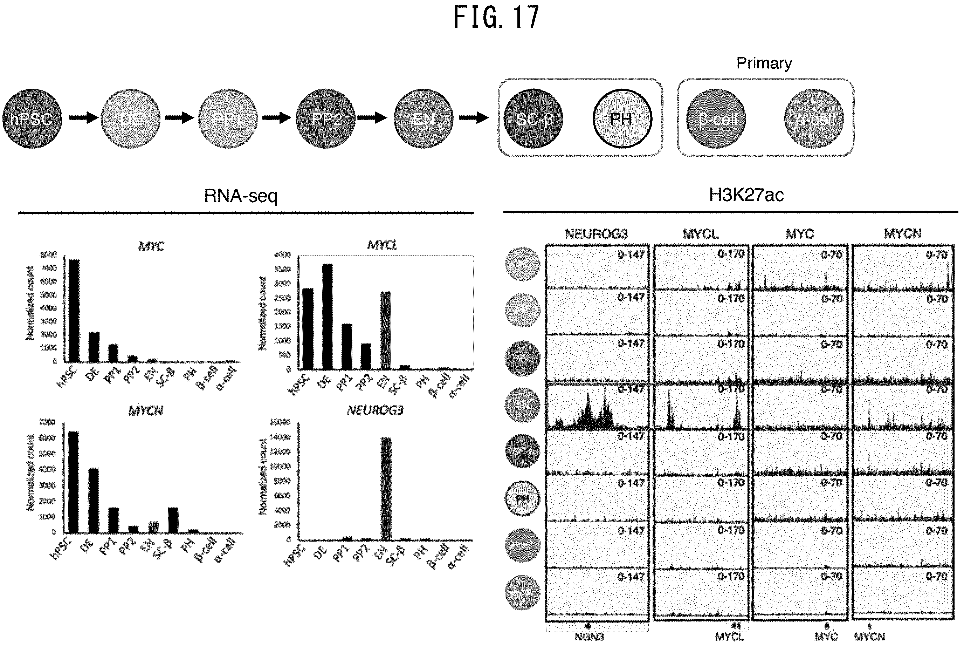

In a paper submitted in 2020 (JR Albarez-Dominguez, et al., Circadian Entry Triggers Measurement of Human In Vitro Islets., Cell Stem Cell, 26 (1), 108-122, 2020), the molecular profile was observed when human pancreatic β-cells were induced to differentiate step by step from human iPS cells. The data set was then used to observe gene expression of the MYC family (described above) and H3K27ac (a chromatin mark generally showing transcriptional activation) (Fig. 17).

Expression of Myc family genes was high in pluripotent stem cells ("hPSC"), and tended to decrease with differentiation, whereas expression of Mycl was transiently elevated in "EN" (endocrine progenitor cells, i.e., pancreatic islet progenitor cells). This suggests that Mycl may contribute to pancreatic islet cell proliferation in human pancreatic islet cells as well as in mice.

### Example 14: Confirmation of pancreatic islet hyperplasia in humans

In human pancreatic islet cells, Mycl and zsGreenDR (control group) were infected with lentiviruses capable of inducing expression in a Dox-dependent manner. The following day, these pancreatic islet cells were transferred to three-dimensional culture using Matrigel and simultaneously administered Dox induced Mycl and zsGreenDR expression, respectively.

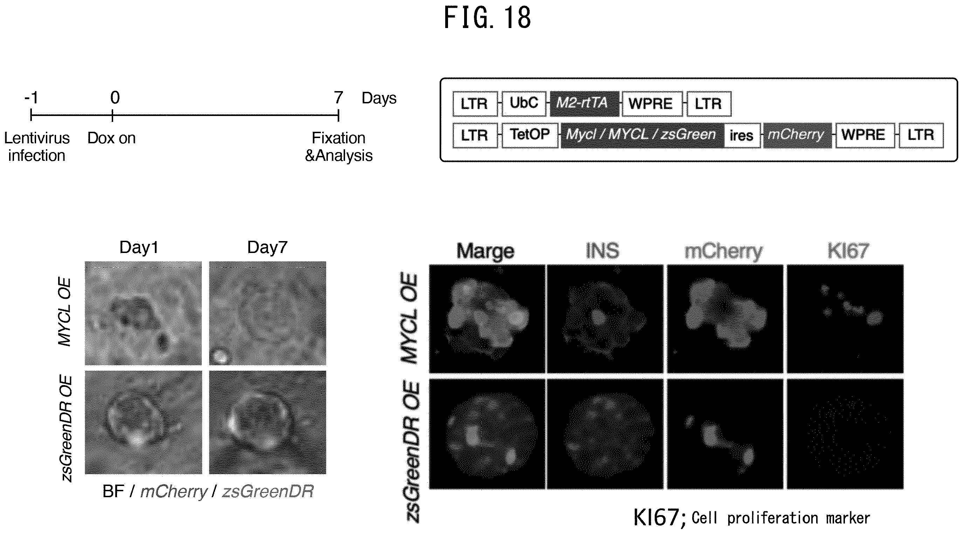

The zsGreenDR-induced pancreatic islet cells showed no change in morphology after 7 days, but an increase in pancreatic islet cells was observed when MYCL was induced (lower left of Fig. 18). Furthermore, immunostaining revealed Ki67 (cell proliferation marker) in cells with mCherry expression (synonymous with MYCL expression) in the MYCL-overexpressing group (lower right side of Fig. 18).

### Example 15: Confirmation of pancreatic islet growth in humans by single-cell analysis

In human pancreatic islet cells, Mycl, MYCL, and zsGreenDR (control group) were infected with lentiviruses capable of inducing expression, respectively. The following day, cells were transferred to suspension cultures using cell culture inserts and at the same time Dox was administered to induce Mycl and zsGreenDR expression, respectively. After 7 days, dead cells were removed and single cell RNA-seq was performed according to routine procedures.

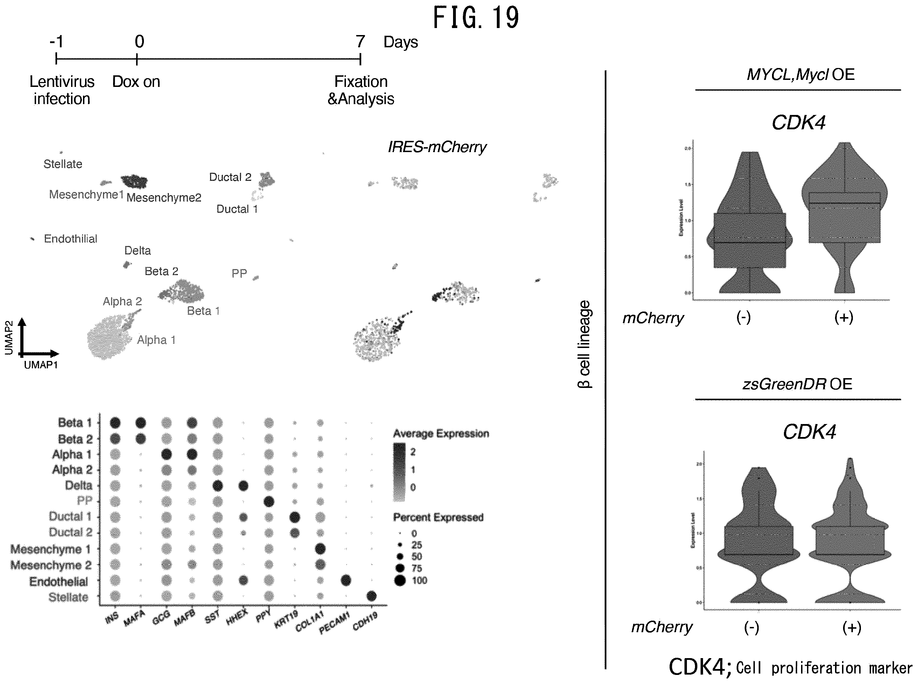

Single cell RNA-seq resulted in the detection of α cells, β cells, δ cells, pancreatic ductal cells ("PP"), mesenchymal cells, vascular endothelial cells, and astrocytes (Fig. 19 left). Analysis of expression of CDK4, a marker of cell proliferation, focused only on β cells, showed that induction of zsGreenDR did not affect CDK4, whereas induction of Mycl and MYCL induced CDK4 expression. It was found that human pancreatic islet cells can also proliferate by inducing Mycl expression.

### INDUSTRIAL APPLICABILITY

The application of the present technology to pancreatic islets isolated from donors allows the proliferation of pancreatic islet insulin-positive cell counts *in vitro* and may eliminate the shortage of pancreatic islet donors in the present invention. The use of this technique in the differentiation induction system of pancreatic islet insulin-positive cells from ES/iPS cells enables efficient differentiation of pancreatic islet insulin-positive cells from pluripotent stem cells. In addition, it is possible to increase the number of insulin-positive re-enrichment in the produced organs by applying the technique to produce pancreatic islets from pluripotent stem cells by blastocyst complementation. The present invention needs to transiently express Mycl on pancreatic pancreatic islet cells derived from donors and pancreatic pancreatic islet cells derived from pluripotent stem cells, but it is also possible to establish a large amount of pancreatic pancreatic islet cells without genetic modification by introducing episomal vectors, RNA, etc. Pancreatic islet cells grown using this technique are expected to proliferate and maintain as pancreatic islet stock cell lines. Pancreatic islet stock cell lines may provide long-term, stable, and large quantities of insulin-positive cells. It is hoped that in the future, it will be possible to provide inexpensive and high-quality pancreatic islets for many diabetic patients.

All publications and patent documents cited herein are hereby incorporated by reference in their entirety. Although specific embodiments of the invention have been described herein for illustrative purposes, it will be readily appreciated by those skilled in the art that various modifications may be made without departing from the spirit and scope of the invention.

## Claims

1. An insulin production promoting agent that includes the Mycl gene or its gene product.

2. The insulin production promoting agent according to claim 1, wherein the Mycl gene includes:
(1) a nucleic acid including the nucleotide sequence represented by SEQ ID NO: 1 or 3; or
(2) a nucleic acid that hybridizes with a nucleic acid including the nucleotide sequence represented by SEQ ID NO: 1 or 3 under stringent conditions and encodes a polypeptide having activity of promoting insulin production when its expression is induced by the Mycl gene.

3. The insulin production promoting agent according to claim 1, wherein the Mycl gene product includes:
(1) a polypeptide including the amino acid sequence represented by SEQ ID NO: 2 or 4; or
(2) a polypeptide having at least 80%, 85%, 90%, 95%, 97%, 98% or 99% sequence identity with the amino acid sequence represented by SEQ ID NO: 2 or 4, and having an effect of causing proliferation of pancreatic islet-like cells and/or activity of promoting insulin production.

4. An insulin production promoting agent, having the Mycl gene or its gene product introduced into pancreatic islet cells.

5. The insulin production promoting agent according to any one of claims 1 to 4, wherein the Mycl gene is transiently expressed.

6. The insulin production promoting agent according to claim 4 or 5, wherein the pancreatic islet cells are derived from primary pancreatic islet cells isolated from a pancreas, cultured pancreatic islet cells or stem cells.

7. The insulin production promoting agent according to claim 6, wherein the stem cells are selected from the group consisting of iPS cells, ES cells and somatic stem cells.

8. A pharmaceutical composition for prevention and/or treatment of diabetes or its associated disease, the pharmaceutical composition including
(i) the Mycl gene or its gene product; a vector in which the Mycl gene has been incorporated; and/or pancreatic islet cells in which the Mycl gene or its gene product has been introduced or its expression has been induced, and
(ii) a pharmaceutically acceptable excipient, diluent or carrier.

9. The pharmaceutical composition according to claim 8, wherein the diabetes or its associated disease is selected from among diseases, disorders or symptoms associated with type I diabetes, type II diabetes, impaired glucose tolerance, hyperglycemia, heterolipidemia, obesity and metabolic syndrome.

10. The pharmaceutical composition according to claim 8 or 9, wherein the Mycl gene includes:
(1) a nucleic acid including the nucleotide sequence represented by SEQ ID NO: 1 or 3; or
(2) a nucleic acid that hybridizes with a nucleic acid including the nucleotide sequence represented by SEQ ID NO: 1 or 3 under stringent conditions and encodes a polypeptide having activity of promoting insulin production when its expression is induced by the Mycl gene.

11. The pharmaceutical composition according to claim 8 or 9, wherein the Mycl gene product includes:
(1) a polypeptide including the amino acid sequence represented by SEQ ID NO: 2 or 4; or
(2) a polypeptide having at least 80%, 85%, 90%, 95%, 97%, 98% or 99% sequence identity with the amino acid sequence represented by SEQ ID NO: 2 or 3, and having an effect of causing proliferation of pancreatic islet-like cells and/or activity of promoting insulin production.

12. A kit including an insulin production promoting agent according to any one of claims 1 to 7 or a pharmaceutical composition according to any one of claims 8 to 11.

13. A kit further including an activator for activation of the Mycl gene.

14. The kit according to claim 13, wherein the activator for activation of the Mycl gene is selected from the group consisting of promoters, enhancers, promoter-activating enzymes or factors, enhancer-activating enzymes or factors, nucleic acid-protein complexes and low molecular compounds.

15. Pancreatic islet cells in which the Mycl gene or its gene product has been introduced.

16. The pancreatic islet-like cells according to claim 15, wherein the pancreatic islet cells are derived from primary pancreatic islet cells isolated from a pancreas, cultured pancreatic islet cells or stem cells.

17. A method for preparing pancreatic islet-like cells according to claim 16, the method including:
(a) a step of incorporating the Mycl gene into a recombination plasmid, recombination viral vector, minicircle or episomal vector; and
(b) a step of introducing the recombination plasmid, recombination viral vector, minicircle or episomal vector obtained in step (a) into pancreatic islet cells.

18. A method for preparing pancreatic islet-like cells according to claim 16, the method including a step of introducing RNA encoding the Mycl gene, or Mycl protein, into pancreatic islet cells.

19. A method for proliferating pancreatic islet-like cells according to claim 15 or 16, or pancreatic islet-like cells prepared by the method of according to claim 17 or 18, the method including a step of expressing the Mycl gene.

20. The method according to claim 19, wherein expression of the Mycl gene is transient.


SEQUENCE LISTING
<110> University of Tokyo

<120> Induction of proliferation of islet cells and differentiation
      into insulin positive cells by transient expression of Mycl

<130> P200628WO

<150> JP2019-223953
<151> 2019-12-11

<160> 18

<170> Patent-In version 3.5

<210> 1
<211> 1107
<212> DNA
<213> Mouse

<400> 1
atggacttcg actcgtatca gcactatttc tacgactatg actgcggaga ggatttctac    60
cgctccacgg cgcccagcga ggacatctgg aagaaattcg agctggtgcc gtcgcccccc   120
acgtcgccgc cctggggctc cggtcccggc gccgtggacc cagcctctgg gattaatccc   180
ggggagccgt ggcctggagg gggtgccggg gacgaggcgg aatctcgggg ccattcgaaa   240
gcctggggca ggaattatgc ttccatcatt cgccgtgact gcatgtggag cggcttctcc   300
gcccgagaac ggctggagag agtggtgagc gacaggctgg ccccaggcgc gccccggggg   360
aacccgccca aagcgcccgc taccccggac ggcactccta gtctggaagc cagtaacccg   420
gcgcccgcca cccaatgtca gctgggcgag cccaagactc aggcctgctc cgggtccgag   480
agccccagcg attctgaagg tgaagagatt gacgtggtga ccgtggagaa gaggcgatct   540
ctggacatcc gaaagccagt caccatcacg gtgcgagcag accccctgga cccctgcatg   600
aagcacttcc atatctctat ccaccaacag cagcataact atgctgcccg ttttcctcca   660
gaaagttgct ctcaagaggg ggatcctgag ccaggtcccc aggaagaggc tccggagata   720
gaagctccca aggagaaaga ggaggaggaa gaggaagagg aggaagaaga gattgtgagc   780
cccccacctg tcggaagtga ggctccccag tcctgccacc ccaaacctgt cagttctgac   840
actgaggacg tgaccaagag gaagaaccat aacttcttgg aacgaaaaag gaggaatgac   900
ctccgctccc ggttcctagc cctgcgggac caggttccca ccctggccag ctgctctaag   960
gcccccaaag tcgtgatcct cagcaaggcg ttagaatact tgcaggcttt ggtgggggct  1020
gaaaagaaaa tggctacaga gaaaaggcag ctccggtgtc ggcaacagca actgcaaaag  1080
agaatcgcgt acctcagtgg ctactaa                                      1107

<210> 2
<211> 368
<212> RPT
<213> Mouse

<400> 2
Met Asp Phe Asp Ser Tyr Gln His Tyr Phe Tyr Asp Tyr Asp Cys Gly
1               5                   10                  15
Glu Asp Phe Tyr Arg Ser Thr Ala Pro Ser Glu Asp Ile Trp Lys Lys
            20                  25                  30
Phe Glu Leu Val Pro Ser Pro Pro Thr Ser Pro Pro Trp Gly Ser Gly
        35                  40                  45
Pro Gly Ala Val Asp Pro Ala Ser Gly Ile Asn Pro Gly Glu Pro Trp
    50                  55                  60
Pro Gly Gly Gly Ala Gly Asp Glu Ala Glu Ser Arg Gly His Ser Lys
65                  70                  75                  80
Ala Trp Gly Arg Asn Tyr Ala Ser Ile Ile Arg Arg Asp Cys Met Trp
                85                  90                  95
Ser Gly Phe Ser Ala Arg Glu Arg Leu Glu Arg Val Val Ser Asp Arg
            100                 105                 110
Leu Ala Pro Gly Ala Pro Arg Gly Asn Pro Pro Lys Ala Pro Ala Thr
        115                 120                 125

Pro Asp Gly Thr Pro Ser Leu Glu Ala Ser Asn Pro Ala Pro Ala Thr
    130                 135                 140
Gln Cys Gln Leu Gly Glu Pro Lys Thr Gln Ala Cys Ser Gly Ser Glu
145                 150                 155                 160
Ser Pro Ser Asp Ser Glu Gly Glu Glu Ile Asp Val Val Thr Val Glu
                165                 170                 175
Lys Arg Arg Ser Leu Asp Ile Arg Lys Pro Val Thr Ile Thr Val Arg
            180                 185                 190
Ala Asp Pro Leu Asp Pro Cys Met Lys His Phe His Ile Ser Ile His
        195                 200                 205
Gln Gln Gln His Asn Tyr Ala Ala Arg Phe Pro Pro Glu Ser Cys Ser
    210                 215                 220
Gln Glu Gly Asp Pro Glu Pro Gly Pro Gln Glu Glu Ala Pro Glu Ile
225                 230                 235                 240
Glu Ala Pro Lys Glu Lys Glu Glu Glu Glu Glu Glu Glu Glu Glu Glu
                245                 250                 255
Glu Ile Val Ser Pro Pro Pro Val Gly Ser Glu Ala Pro Gln Ser Cys
            260                 265                 270
His Pro Lys Pro Val Ser Ser Asp Thr Glu Asp Val Thr Lys Arg Lys
        275                 280                 285
Asn His Asn Phe Leu Glu Arg Lys Arg Arg Asn Asp Leu Arg Ser Arg
    290                 295                 300
Phe Leu Ala Leu Arg Asp Gln Val Pro Thr Leu Ala Ser Cys Ser Lys
305                 310                 315                 320
Ala Pro Lys Val Val Ile Leu Ser Lys Ala Leu Glu Tyr Leu Gln Ala
                325                 330                 335
Leu Val Gly Ala Glu Lys Lys Met Ala Thr Glu Lys Arg Gln Leu Arg
            340                 345                 350
Cys Arg Gln Gln Gln Leu Gln Lys Arg Ile Ala Tyr Leu Ser Gly Tyr
        355                 360                 365

<210> 3
<211> 1095
<212> DNA
<213> Human

<400> 3
atggactacg actcgtacca gcactatttc tacgactatg actgcgggga ggatttctac    60
cgctccacgg cgcccagcga ggacatctgg aagaaattcg agctggtgcc atcgcccccc   120
acgtcgccgc cctggggctt gggtcccggc gcaggggacc cggcccccgg gattggtccc   180
ccggagccgt ggcccggagg gtgcaccgga gacgaagcgg aatcccgggg ccactcgaaa   240
ggctggggca ggaactacgc ctccatcata cgccgtgact gcatgtggag cggcttctcg   300
gcccgggaac ggctggagag agctgtgagc gaccggctcg ctcctggcgc gccccggggg   360
aacccgccca aggcgtccgc cgccccggac tgcactccca gcctcgaagc cggcaacccg   420
gcgcccgccg ccccctgtcc gctgggcgaa cccaagaccc aggcctgctc cgggtccgag   480
agcccaagcg actcggagaa tgaagaaatt gatgttgtga cagtagagaa gaggcagtct   540
ctgggtattc ggaagccggt caccatcacg gtgcgagcag accccctgga tccctgcatg   600
aagcatttcc acatctccat ccatcagcaa cagcacaact atgctgcccg ttttcctcca   660
gaaagctgct cccaagaaga ggcttcagag aggggtcccc aagaagaggt tctggagaga   720
gatgctgcag gggaaaagga agatgaggag gatgaagaga ttgtgagtcc cccacctgta   780
gaaagtgagg ctgcccagtc ctgccacccc aaacctgtca gttctgatac tgaggatgtg   840
accaagagga agaatcacaa cttcctggag cgcaagaggc ggaatgacct gcgttcgcga   900
ttcttggcgc tgagggacca ggtgcccacc ctggccagct gctccaaggc ccccaaagta   960
gtgatcctaa gcaaggcctt ggaatacttg caagccctgg tgggggctga gaagaggatg  1020
gctacagaga aaagacagct ccgatgccgg cagcagcagt tgcagaaaag aattgcatac  1080
ctcactggct actaa                                                  1095


<210> 4
<211> 364
<213> Human

<400> 4

Met Asp Tyr Asp Ser Tyr Gln His Tyr Phe Tyr Asp Tyr Asp Cys Gly
1               5                   10                  15
Glu Asp Phe Tyr Arg Ser Thr Ala Pro Ser Glu Asp Ile Trp Lys Lys
            20                  25                  30
Phe Glu Leu Val Pro Ser Pro Pro Thr Ser Pro Pro Trp Gly Leu Gly
        35                  40                  45
Pro Gly Ala Gly Asp Pro Ala Pro Gly Ile Gly Pro Pro Glu Pro Trp
    50                  55                  60
Pro Gly Gly Cys Thr Gly Asp Glu Ala Glu Ser Arg Gly His Ser Lys
65                  70                  75                  80
Gly Trp Gly Arg Asn Tyr Ala Ser Ile Ile Arg Arg Asp Cys Met Trp
                85                  90                  95
Ser Gly Phe Ser Ala Arg Glu Arg Leu Glu Arg Ala Val Ser Asp Arg
            100                 105                 110
Leu Ala Pro Gly Ala Pro Arg Gly Asn Pro Pro Lys Ala Ser Ala Ala
        115                 120                 125
Pro Asp Cys Thr Pro Ser Leu Glu Ala Gly Asn Pro Ala Pro Ala Ala
    130                 135                 140
Pro Cys Pro Leu Gly Glu Pro Lys Thr Gln Ala Cys Ser Gly Ser Glu
145                 150                 155                 160
Ser Pro Ser Asp Ser Glu Asn Glu Glu Ile Asp Val Val Thr Val Glu
                165                 170                 175
Lys Arg Gln Ser Leu Gly Ile Arg Lys Pro Val Thr Ile Thr Val Arg
            180                 185                 190
Ala Asp Pro Leu Asp Pro Cys Met Lys His Phe His Ile Ser Ile His
        195                 200                 205
Gln Gln Gln His Asn Tyr Ala Ala Arg Phe Pro Pro Glu Ser Cys Ser
    210                 215                 220
Gln Glu Glu Ala Ser Glu Arg Gly Pro Gln Glu Glu Val Leu Glu Arg
225                 230                 235                 240
Asp Ala Ala Gly Glu Lys Glu Asp Glu Glu Asp Glu Glu Ile Val Ser
                245                 250                 255
Pro Pro Pro Val Glu Ser Glu Ala Ala Gln Ser Cys His Pro Lys Pro
            260                 265                 270
Val Ser Ser Asp Thr Glu Asp Val Thr Lys Arg Lys Asn His Asn Phe
        275                 280                 285
Leu Glu Arg Lys Arg Arg Asn Asp Leu Arg Ser Arg Phe Leu Ala Leu
    290                 295                 300
Arg Asp Gln Val Pro Thr Leu Ala Ser Cys Ser Lys Ala Pro Lys Val
305                 310                 315                 320
Val Ile Leu Ser Lys Ala Leu Glu Tyr Leu Gln Ala Leu Val Gly Ala
                325                 330                 335
Glu Lys Arg Met Ala Thr Glu Lys Arg Gln Leu Arg Cys Arg Gln Gln
            340                 345                 350
Gln Leu Gln Lys Arg Ile Ala Tyr Leu Thr Gly Tyr
        355                 360

<210> 5
<211> 20
<212> DNA
<213> Artificial

<220>
<223> Forward primer sequence for amplifying beta-actin gene

<400> 5
gccaaccgtg aaaagatgac                                                60

<210> 6
<211> 19
<212> DNA
<213> Artificial

<220>
<223> Reverse primer sequence for amplifying beta-actin gene

<400> 6
tccggagtcc atcacaatg                                                    19

<210> 7
<211> 18
<212> DNA
<213> Artificial

<220>
<223> Forward primer sequence for amplifying Myc gene

<400> 7
caccagcagc gactctga                                                     18

<210> 8
<211> 18
<212> DNA
<213> Artificial

<220>
<223> Reverse primer sequence for amplifying Myc gene

<400> 8
ggggtttgcc tcttctcc                                                     18

<210> 9
<211> 20
<212> DNA
<213> Artificial

<220>
<223> Forward primer sequence for amplifying Mycn gene

<400> 9
ctccggagag gataccttga                                                   20

<210> 10
<211> 20
<212> DNA
<213> Artificial

<220>
<223> Reverse primer sequence for amplifying Mycn gene

<400> 10
tctctacggt gaccacatcg                                                   20

<210> 11
<211> 20
<212> DNA
<213> Artificial

<220>
<223> Forward primer sequence for amplifying Mycl gene

<400> 11
acggcactcc tagtctggaa                                                   20

<210> 12
<211> 21

<212> DNA
<213> Artificial

<220>
<223> Reverse primer sequence for amplifying Mycl gene

<400> 12
ccacgtcaat ctcttcacct t                                                21

<210> 13
<211> 19
<212> DNA
<213> Artificial

<220>
<223> Forward primer sequence for amplifying Fev gene

<400> 13
ccgtcggaga tggtctttt                                                   19

<210> 14
<211> 20
<212> DNA
<213> Artificial

<220>
<223> Reverse primer sequence for amplifying Fev gene

<400> 14
ccaggagaaa ctgccacaac                                                  20

<210> 15
<211> 20
<212> DNA
<213> Artificial

<220>
<223> Forward primer sequence for amplifying Cck gene

<400> 15
actgctagcg cgatacatcc                                                  20

<210> 16
<211> 20
<212> DNA
<213> Artificial

<220>
<223> Reverse primer sequence for amplifying Cck gene

<400> 16
tattctatgg ctggggtcca                                                  20

<210> 17
<211> 20
<212> DNA
<213> Artificial

<220>
<223> Forward primer sequence for amplifying Pax4 gene

<400> 17
gtacttcggg cacttcagga                                                  20

<210> 18
<211> 20
<212> DNA
<213> Artificial

<220>
<223> Reverse primer sequence for amplifying Pax4 gene

<400> 18
ttactgtggg gactgggaag                                                    20